# EUROPEAN PATENT SPECIFICATION

(11) **EP 3 092 993 B1**
(45) Date of publication and mention of the grant of the patent: **03.01.2018**
(21) Application number: 14877580.2
(22) Date of filing: 09.10.2014
(51) Int. Cl.: B29C 65/08, A61F 13/15, A61F 13/49, B29C 65/78, B29L 31/48

(54) **ULTRASONIC WELDING APPARATUS AND ULTRASONIC WELDING METHOD OF SHEET-LIKE MEMBER ASSOCIATED WITH ABSORBENT ARTICLE**
ULTRASCHALLSCHWEISSVORRICHTUNG UND ULTRASCHALLSCHWEISSVERFAHREN FÜR BAHNENFÖRMIGE WERKSTÜCKE IN VERBINDUNG MIT SAUGFÄHIGEN ARTIKELN
DISPOSITIF DE SOUDAGE ULTRASONORE ET PROCÉDÉ DE SOUDAGE ULTRASONORE D'ÉLÉMENT EN FORME DE FEUILLE ASSOCIÉ À UN ARTICLE ABSORBANT

(30) Priority: 10.01.2014 JP 2014002990
(43) Date of publication of application: 16.11.2016
(73) Proprietor: Unicharm Corporation, Ehime 799-0111 (JP)
(72) Inventor: YAMAMOTO, Hiroki, Kanonji-shi Kagawa 769-1602 (JP); MATSUMOTO, Yoshihiko, Kanonji-shi Kagawa 769-1602 (JP)
(74) Representative: Dolleymores
(86) International application number: PCT/JP2014/077097
(87) International publication number: WO 2015/104879

(56) References cited:
- WO-A1-2012/042842
- WO-A1-2013/141022
- CN-A- 1 179 128
- JP-A- H0 857 959
- JP-A- H10 291 082
- JP-A- H10 513 128
- JP-A- 2000 015 701
- JP-A- 2010 094 883
- JP-A- 2013 233 729
- US-A- 5 643 396
- US-A- 5 667 608

## Description

### [Technical Field]

The invention relates to an ultrasonic welding apparatus and an ultrasonic welding method of welding a sheet-like member using ultrasonic vibration, the sheet-like member being associated with an absorbent article such as a disposable diaper.

### [Background Art]

Conventionally, in a production line of an absorbent article such as a disposable diaper, a sheet-like member 1a, which is configured with a plurality of stacked continuous sheets (e.g. nonwoven fabric), is welded so that these continuous sheets are joined. As an example of an apparatus 120 which performs such a welding process, Patent Literature 1 discloses an ultrasonic welding apparatus 120. The apparatus 120 generates friction heat by applying ultrasonic energy to the sheet-like member 1a, and thereby welds the sheet-like member 1a.

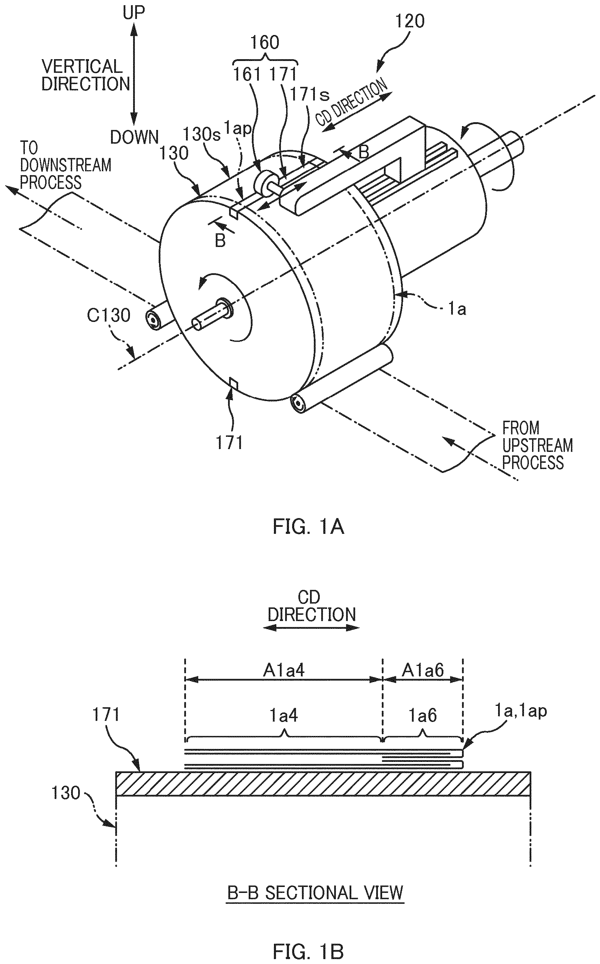

FIG. 1A is a schematic perspective view of the apparatus 120. The apparatus 120 includes a rotating drum 130 which rotates about a central axis C130. A sheet-like member 1a to be welded (see double-dotted chain lines in FIG. 1A) is wound around the outer circumferential face 130s of the rotating drum 130, and the rotating drum 130 rotates about the central axis C130. Accordingly, the sheet-like member 1a is substantially integrated with the outer circumferential face 130s of the rotating drum 130 and is transported.

The apparatus 120 also includes an ultrasonic processing unit 160, and the unit 160 rotates about the central axis C130 in an integrated manner with the rotating drum 130. During the rotation, the unit 160 performs ultrasonic welding process to the sheet-like member 1a.

That is, the ultrasonic processing unit 160 includes a rail-like anvil 171 and a roller-like horn 161 on the outer circumferential face 130s of the rotating drum 130. The anvil 171 extends in the CD direction, which is orthogonal to the transport direction. The horn 161 vibrates ultrasonically and is provided outside with respect to the anvil 171 in the rotation-radius direction of the rotating drum 130. During a period in which the horn 161 and the anvil 171 both are rotating about the central axis C130 together with the rotating drum 130 and the sheet-like member 1a, the roller-like horn 161 rolls on the anvil 171 along the CD direction and reciprocates in the CD direction. At this time, the horn 161 applies ultrasonic energy to a part 1ap of the sheet-like member 1a which is placed on the anvil 171, and thereby welds the part 1ap.

### [Citation List]

### [Patent Literature].

[Patent Literature 1] Japanese Unexamined Patent Application Publication (Translation of PCT Application) No. 10-513128. US 5667608 A and EP0807013 A1 are patent family members. WO 2013/141022 A1, WO 2012/042842 A1, CN 1179128 A and US 5643396 A also relate to ultrasonic processing systems for webs.

### [Summary of Invention]

### [Technical Problem]

However, Patent Literature 1 does not disclose an amount of ultrasonic energy applied during reciprocation.

Here, if the amount (J/m) of ultrasonic energy applied per unit length in the CD direction is made different between sections A1a4 and A1a6 whose positions are different from each other in the CD direction, which can solve a predetermined problem, it would be convenient. FIG. 1B is an explanatory diagram of the above description, and is the schematic cross-sectional view (cross-sectional view taken along line B-B of FIG. 1A) illustrating a structure in the thickness direction of the sheet-like member 1a.

For example, as illustrated in FIG. 1B, in the case where the number of stacked continuous sheets in the sheet-like member 1a is different depending on the position in the CD direction, the amount (J/m) of ultrasonic energy necessary for welding of the sheet-like member 1a results in being different depending on the position in the CD direction. Specifically, when a part 1a6, in which the number of stacked sheets is six, and a part 1a4, in which the number of stacked sheets is four, exist in the CD direction, the amount (J/m) of ultrasonic energy necessary for welding of the six-sheet part 1a6 is greater than the amount (J/m) of ultrasonic energy necessary for welding of the four-sheet part 1a4.

Thus, if the amount (J/m) of ultrasonic energy cannot be changed according to the positions in the CD direction, the ultrasonic energy cannot help being applied with the amount (J/m) suitable for either one of the four-sheet part 1a4 and the six-sheet part 1a6.

However, in such a case, if the six-sheet part 1a6 is welded with the amount (J/m) of ultrasonic energy for the four-sheet part 1a4, the applied ultrasonic energy may be insufficient, which may cause a joint failure. On the other hand, if the four-sheet part 1a4 is welded with the amount (J/m) of ultrasonic energy for the six-sheet part 1a6, the applied ultrasonic energy may be excessive, the part 1a4 may be melted resulting in a loss thereof.

Such problems can be solved, if the amount (J/m) of ultrasonic energy can be made different between the sections A1a4 and A1a6 set at positions different from each other in the CD direction.

Further, even if the number of stacked continuous sheets configuring the sheet-like member 1a is the same throughout the CD direction, it may be desired that the strength of welding is made different according to the positions in the CD direction. In such a case, the amount (J/m) of ultrasonic energy applied per unit length in the CD direction may be increased in a part in which weld strength is to be made higher in the sheet-like member 1a. On the other hand, the amount (J/m) of applied ultrasonic energy per unit length in the CD direction may be reduced in a part in which weld strength is to be made lower.

The present invention is to be made in view of the above described conventional problems, and an object thereof directed to provide an ultrasonic welding apparatus and an ultrasonic welding method which can preferably be used when the amount (J/m) of ultrasonic energy necessary for welding is different from each other between the first section and the second section whose positions in the CD direction are different from each other.

### [Solution to Problem]

The present invention provides the ultrasonic welding apparatus of independent claim 1 and the ultrasonic welding method of independent claim 12. The dependent claims specify preferred but optional features. An aspect of the invention to achieve the above advantage is an ultrasonic welding apparatus that performs an ultrasonic welding process to a sheet-like member, the sheet-like member being associated with an absorbent article, the sheet-like member being transported while being wound onto an outer circumferential face of a rotating member, the rotating member rotating about its central axis, the apparatus comprising:
the rotating member;
an ultrasonic processing unit
   that rotates about the central axis together with the rotating member, and
   that includes, as ultrasonic processing members,
      a horn that vibrates ultrasonically and
      an anvil between which and the horn the sheet-like member is sandwiched; and
an ultrasonic-energy regulating unit,
at least either one ultrasonic processing member of the horn and the anvil being guided so as to reciprocate in a CD direction, the CD direction intersecting a transport direction of the sheet-like member,
the at least either one ultrasonic processing member of the horn and the anvil reciprocating in the CD direction with respect to a part of the sheet-like member, the part being wound around the rotating member,
the outer circumferential face of the rotating member including a first section and a second section at positions different from each other in the CD direction,
the ultrasonic-energy regulating unit making
   an amount (J/m) of ultrasonic energy applied per unit length in the CD direction when the ultrasonic processing member passes through the first section on either one of a forward path and a return path of the reciprocation
   different from
   an amount (J/m) of ultrasonic energy applied per unit length in the CD direction when the ultrasonic processing member passes through the second section on either one of the forward path and the return path.

Further,
an ultrasonic welding method in which a sheet-like member associated with an absorbent article is subject to an ultrasonic welding process, the sheet-like member being transported while being wound onto an outer circumferential face of a rotating member, the rotating member rotating about its central axis, the method comprising:
rotating an ultrasonic processing unit about the central axis together with the rotating member, the ultrasonic processing unit including, as ultrasonic processing members, a horn and an anvil facing the horn;
causing the horn to vibrate ultrasonically; and
causing at least either one ultrasonic processing member of the horn and the anvil to reciprocate in a CD direction with respect to a part of the sheet-like member, the CD direction intersecting a transport direction of the sheet-like member, while the sheet-like member is sandwiched between the horn and the anvil, the part being wound around the rotating member,
the outer circumferential face of the rotating member including a first section and a second section at positions different from each other in the CD direction,
an amount (J/m) of ultrasonic energy applied per unit length in the CD direction when the ultrasonic processing member passes through the first section on either one of a forward path and a return path of the reciprocation being made different from an amount (J/m) of ultrasonic energy applied per unit length in the CD direction when the ultrasonic processing member passes through the second section on either one of the forward path and the return path.

Other features of this invention will become apparent from the description in this specification and the attached drawings.

### [Advantageous Effects of Invention]

According to the invention, an ultrasonic welding apparatus and an ultrasonic welding method can be provided, which can preferably be used when an amount (J/m) of ultrasonic energy necessary for welding is different from each other between a first section and a second section whose positions are different from each other in the CD direction.

### [Brief Description of Drawings]

FIG. 1A is a schematic perspective view of a conventional ultrasonic welding apparatus 120. FIG. 1B is a cross-sectional view taken along line B-B of FIG. 1A.
FIG. 2A is a schematic perspective view of a substrate 1a of a diaper 1 which has not two-folded yet. FIG. 2B is a schematic perspective view of the two-folded substrate 1a immediately before being transported to an ultrasonic welding apparatus 20 according to the first embodiment. FIG. 2C is a cross-sectional view taken along line C-C of FIG. 2A. FIG. 2D is a cross-sectional view taken along line D-D of FIG. 2B.
FIG. 3 is a schematic perspective view of the ultrasonic welding apparatus 20 of the first embodiment as viewed obliquely from front above.
FIG. 4 is a schematic side view along arrows IV-IV in FIG. 3.
FIG. 5 is a schematic front view along arrows V-V in FIG. 3.
FIG. 6 is a partially cutaway, schematic side view with the substrate 1a and a rotating drum 30 being removed from FIG. 4.
FIG. 7A is a schematic perspective view of a support unit 73 as viewed obliquely from front and outside in a rotation-radius direction Dr30. FIG. 7B is a schematic perspective view of the support unit 73 as viewed obliquely from back and outside in the rotation-radius direction Dr30.
FIG. 8 is a schematic front view of the ultrasonic welding apparatus 20 and illustrates the ranges in a rotation direction Dc30 to which a forward movement and a backward movement are assigned, the forward movement being a movement in a forward path, the backward movement being a movement in a return path.
FIG. 9 is a schematic perspective view of an anvil roller 71.
FIG. 10A is a schematic diagram illustrating a relative positional relationship between a horn 61 and the substrate 1a wound onto the outer circumferential face 30s of the rotating drum 30. FIG. 10B is a cross-sectional diagram taken along line B-B of FIG. 10A.
FIG. 11A is a conceptual diagram illustrating a problem that a weld residue created at an end edge part 1ae1 in the CD direction of the substrate 1a. FIG. 11B is a conceptual diagram illustrating a solution thereof that such a weld residue is created in the center in the CD direction of the substrate 1a
FIG. 12A is a schematic diagram of an ultrasonic processing unit 60a according to the second modified example as viewed in the rotation direction Dc30 of the rotating drum 30. FIG. 12B is a schematic front view of the ultrasonic welding apparatus 20, and is the view for explaining a cam curve according to the third modified example.
FIG. 13 is a schematic perspective view of an ultrasonic welding apparatus 20b according to the second embodiment as viewed obliquely from front above.
FIG. 14 is a schematic side view along arrows XIV-XIV in FIG. 13.
FIG. 15 is a schematic front view along arrows XV-XV in FIG. 13.
FIG. 16 is a schematic side view obtained by removing the substrate 1a and the rotating drum 30 from FIG. 14.
FIG. 17A is a schematic perspective view of an ultrasonic processing unit 60b according to the second embodiment as viewed obliquely from front and outside in the rotation-radius direction Dr30, and FIG. 17B is a schematic perspective view of the unit 60b as viewed obliquely from back and outside.
FIG. 18 is a schematic perspective view of an ultrasonic processing unit 60c showing another example of the positional relationship between a horn 61b and the anvil roller 71 according to the second embodiment.
FIG. 19A is a schematic plan view of an example in which a plurality of parts 1P1BH, 1P1C, 1P1LH, having weld strength different from each other, in each of side end parts 1e in the width direction of the diaper 1. FIG. 19B is a schematic diagram illustrating a state in which the substrate 1a of the diaper 1 is wound onto the rotating drum 30 of the ultrasonic welding apparatus 20.

### [Description of Embodiments]

At least the following matters will be made clear by the description in the present specification and the accompanying drawings.

An ultrasonic welding apparatus that performs an ultrasonic welding process to a sheet-like member, the sheet-like member being associated with an absorbent article, the sheet-like member being transported while being wound onto an outer circumferential face of a rotating member, the rotating member rotating about its central axis, the apparatus comprising:
the rotating member;
an ultrasonic processing unit
   that rotates about the central axis together with the rotating member, and
   that includes, as ultrasonic processing members,
      a horn that vibrates ultrasonically and
      an anvil between which and the horn the sheet-like member is sandwiched; and
an ultrasonic-energy regulating unit,
at least either one ultrasonic processing member of the horn and the anvil being guided so as to reciprocate in a CD direction, the CD direction intersecting a transport direction of the sheet-like member,
the at least either one ultrasonic processing member of the horn and the anvil reciprocating in the CD direction with respect to a part of the sheet-like member, the part being wound around the rotating member,
the outer circumferential face of the rotating member including a first section and a second section at positions different from each other in the CD direction,
the ultrasonic-energy regulating unit making
   an amount (J/m) of ultrasonic energy applied per unit length in the CD direction when the ultrasonic processing member passes through the first section on either one of a forward path and a return path of the reciprocation
   different from
   an amount (J/m) of ultrasonic energy applied per unit length in the CD direction when the ultrasonic processing member passes through the second section on either one of the forward path and the return path.

According to such an ultrasonic welding apparatus for a sheet-like member associated with an absorbent article, the amount (J/m) of applied ultrasonic energy is made different from each other between the first section and the second section whose positions in the CD direction are different from each other. Thus, it is possible to appropriately perform a welding process to a sheet-like member in which the amount (J/m) of ultrasonic energy necessary for welding is different from each other between a part corresponding to the first section and a part corresponding to the second section.

In such an ultrasonic welding apparatus for a sheet-like member associated with an absorbent article, it is preferable that when the ultrasonic processing member passes through the first section and the second section on one of the forward path and the return path of the reciprocation, a first part corresponding to the first section in the sheet-like member is welded by applying an amount (J/m) of the ultrasonic energy corresponding to the first part, and a second part corresponding to the second section in the sheet-like member is welded by applying an amount (J/m) of the ultrasonic energy corresponding to the second part.

According to such an ultrasonic welding apparatus for a sheet-like member associated with an absorbent article, the amount (J/m) of ultrasonic energy necessary for welding is applied to the first section and the second section, on one of the forward path and the return path. Thus, the ultrasonic energy necessary for welding of the first section and the ultrasonic energy necessary for welding of the second section can effectively be restrained from being doubly applied to a part in the first section or the second section.

In such an ultrasonic welding apparatus for a sheet-like member associated with an absorbent article, it is preferable that when the ultrasonic processing member passes through the first section on one of the forward path and the return path of the reciprocation, a first part corresponding to the first section in the sheet-like member is welded by applying an amount (J/m) of the ultrasonic energy corresponding to the first part, when the ultrasonic processing member passes through the second section on an other of the forward path and the return path of the reciprocation, a second part corresponding to the second section in the sheet-like member is welded by applying an amount (J/m) of the ultrasonic energy corresponding to the second part, and on the forward path, the ultrasonic processing member passes through the first section and thereafter the second section, and the second part is not welded in the second section on the forward path, on the return path, the ultrasonic processing member passes through the second section and thereafter the first section, and the first part is not welded in the first section on the return path.

According to such an ultrasonic welding apparatus for a sheet-like member associated with an absorbent article, when the ultrasonic processing member passes through the first section on the forward path, the first part of the sheet-like member is welded. Whereas, in the second section thorough which the ultrasonic processing member passes thereafter on the same forward path, the second part of the sheet-like member is not welded. Thus, on the forward path, weld residue is reliably prevented from protruding from an end edge part in the CD direction of the sheet-like member and remaining. Similarly, when the ultrasonic processing member passes through the second section on the return path, the second part of the sheet-like member is welded. Whereas, in the first section through which the ultrasonic processing member passes thereafter on the same return path, the first part of the sheet-like member is not welded. Thus, also on the return path, weld residue is reliably prevented from protruding from an end edge part in the CD direction of the sheet-like member and remaining.

In such an ultrasonic welding apparatus for a sheet-like member associated with an absorbent article, it is preferable that the sheet-like member includes a plurality of sheets stacked in a thickness direction, the sheet-like member includes a plurality of parts having the number of stacked sheets different from each other, the plurality of parts being arranged in the CD direction, and assuming that a part having the greater number of stacked sheets in the plurality of parts is a first part, and a part having the number of stacked sheets smaller than the number thereof in the first part is a second part, the first section corresponds to the first part, and the second section corresponds to the section part, and an amount (J/m) of the ultrasonic energy applied per unit length in the first section is greater than an amount (J/m) of the ultrasonic energy applied per unit length in the second section.

According to such an ultrasonic welding apparatus for a sheet-like member associated with an absorbent article, the amount (J/m) of ultrasonic energy applied to the first part having the greater number of stacked sheets is set greater than the amount (J/m) of ultrasonic energy applied to the second part having the smaller number of stacked sheets. Thus, the suitable amounts (J/m) of ultrasonic energy for welding of the first part and the second part can be applied to the first part and the second part, respectively. As a result, a joint failure caused by the insufficient application of ultrasonic energy to the first part, a melting loss caused by excessive application of ultrasonic energy to the second part, and the like can effectively restrained.

In such an ultrasonic welding apparatus for a sheet-like member associated with an absorbent article, it is preferable that the sheet-like member includes a plurality of parts between or among which weld strength is to be made different from each other, the plurality of parts being arranged in the CD direction, and assuming that a part that is to have greater weld strength in the plurality of parts is a first part, and a part that is to have weld strength smaller than the weld strength in the first part is a second part, the first section corresponds to the first part, and the second section corresponds to the second part, and an amount (J/m) of the ultrasonic energy applied per unit length in the first section is greater than an amount (J/m) of the ultrasonic energy applied per unit length in the second section.

According to such an ultrasonic welding apparatus for a sheet-like member associated with an absorbent article, the greater amount (J/m) of ultrasonic energy is applied to the first part which is necessary for higher weld strength, while the smaller amount (J/m) of ultrasonic energy is applied to the second part which is necessary for lower weld strength. Thus, the weld strength in the first part can be made higher than the weld strength in the second part.

In such an ultrasonic welding apparatus for a sheet-like member associated with an absorbent article, it is preferable that the ultrasonic-energy regulating unit changes an amount (J/m) of the ultrasonic energy applied per unit length, by changing an amplitude of ultrasonic vibration of the horn.

According to such an ultrasonic welding apparatus for a sheet-like member associated with an absorbent article, since the amount (J/m) of ultrasonic energy is changed by changing the amplitude of the ultrasonic vibration, the amount (J/m) of the ultrasonic energy can be changed in an easy and high responsive manner. Thus, the corresponding amounts (J/m) of ultrasonic energy can swiftly be applied to the first section and the second section, respectively.

In such an ultrasonic welding apparatus for a sheet-like member associated with an absorbent article, it is preferable that the ultrasonic-energy regulating unit changes an amount (J/m) of the ultrasonic energy applied per unit length, by changing a magnitude of sandwiching force with which the sheet-like member is sandwiched between the horn and the anvil.

According to such an ultrasonic welding apparatus for a sheet-like member associated with an absorbent article, the amount (J/m) of ultrasonic energy can be changed by changing the magnitude of the abovementioned sandwiching force. Thus, the amount (J/m) of the ultrasonic energy can be changed easily and reliably.

In such an ultrasonic welding apparatus for a sheet-like member associated with an absorbent article, it is preferable that the ultrasonic-energy regulating unit changes an amount (J/m) of the ultrasonic energy applied per unit length, by changing a size of a space between the horn and the anvil.

According to such an ultrasonic welding apparatus for a sheet-like member associated with an absorbent article, the amount (J/m) of ultrasonic energy is changed by changing the size of the space between the horn and the anvil. Thus, the amount (J/m) of the ultrasonic energy can be changed easily and reliably.

In such an ultrasonic welding apparatus for a sheet-like member associated with an absorbent article, it is preferable that the ultrasonic-energy regulating unit changes an amount (J/m) of the ultrasonic energy applied per unit length, by changing a travel speed value at which the ultrasonic processing member moves in the CD direction.

According to such an ultrasonic welding apparatus for a sheet-like member associated with an absorbent article, the amount (J/m) of ultrasonic energy is changed by changing the travel speed value at which the ultrasonic processing member moves in the CD direction. Thus, the amount (J/m) of the ultrasonic energy can reliably be changed.

In such an ultrasonic welding apparatus for a sheet-like member associated with an absorbent article, it is preferable that the ultrasonic processing member that is either one of the horn and the anvil includes a roller member, the roller member being provided rotatably and is arranged outside with respect to the outer circumferential face of the rotating member, and the roller member moves in the CD direction while rolling on a rail-like member, the rail-like member being provided extending in the CD direction not to move relative to the outer circumferential face of the rotating member, the rail-like member serving as the other ultrasonic processing member.

According to such an ultrasonic welding apparatus for a sheet-like member associated with an absorbent article, the rail-like member serving as the other ultrasonic processing member is provided immovably relative to the outer circumferential face of the rotating member. Thus, the other ultrasonic processing member can keep the relative relationship between itself and the sheet-like member wound around the outer circumferential face in a constant state. As a result, the ultrasonic welding process can be stabilized.

In such an ultrasonic welding apparatus for a sheet-like member associated with an absorbent article, it is preferable that both of the horn and the anvil reciprocate in the CD direction while the sheet-like member is being sandwiched between the horn and the anvil.

According to such an ultrasonic welding apparatus for a sheet-like member associated with an absorbent article, both the horn and the anvil move in the CD direction relative to the sheet-like member. Thus, weld residue, which may be adhered to / accumulated onto at least the horn due to the ultrasonic welding process, can sequentially be wiped by coming into contact with the sheet-like member during the reciprocation in the CD direction. As a result, weld residue can effectively be restrained from being accumulated onto the horn.

Further,
an ultrasonic welding method in which a sheet-like member associated with an absorbent article is subject to an ultrasonic welding process, the sheet-like member being transported while being wound onto an outer circumferential face of a rotating member, the rotating member rotating about its central axis, the method comprising:
rotating an ultrasonic processing unit about the central axis together with the rotating member, the ultrasonic processing unit including, as ultrasonic processing members, a horn and an anvil facing the horn;
causing the horn to vibrate ultrasonically; and
causing at least either one ultrasonic processing member of the horn and the anvil to reciprocate in a CD direction with
respect to a part of the sheet-like member, the CD direction intersecting a transport direction of the sheet-like member, while the sheet-like member is sandwiched between the horn and the anvil, the part being wound around the rotating member,
the outer circumferential face of the rotating member including a first section and a second section at positions different from each other in the CD direction,
an amount (J/m) of ultrasonic energy applied per unit length in the CD direction when the ultrasonic processing member passes through the first section on either one of a forward path and a return path of the reciprocation being made different from an amount (J/m) of ultrasonic energy applied per unit length in the CD direction when the ultrasonic processing member passes through the second section on either one of the forward path and the return path.

According to such an ultrasonic welding apparatus for a sheet-like member associated with an absorbent article, the amount (J/m) of applied ultrasonic energy is made different from each other between the first section and the second section whose positions in the CD direction are different from each other. Thus, a welding process can appropriately be performed to the sheet-like member, in which the amount (J/m) of ultrasonic energy necessary for welding is different from each other between the part corresponding to the first section and the part corresponding to the second section.

### === First Embodiment ===

An ultrasonic welding apparatus 20 according to the invention is an apparatus which forms welded parts 14 in a continuous sheet-like member 1a at intervals in the transport direction (e.g. a predetermined pitch P1), the sheet-like member 1a being transported in a production line. In this embodiment, the sheet-like member 1a is exemplified by the substrate 1a of pull-on disposable diapers 1.

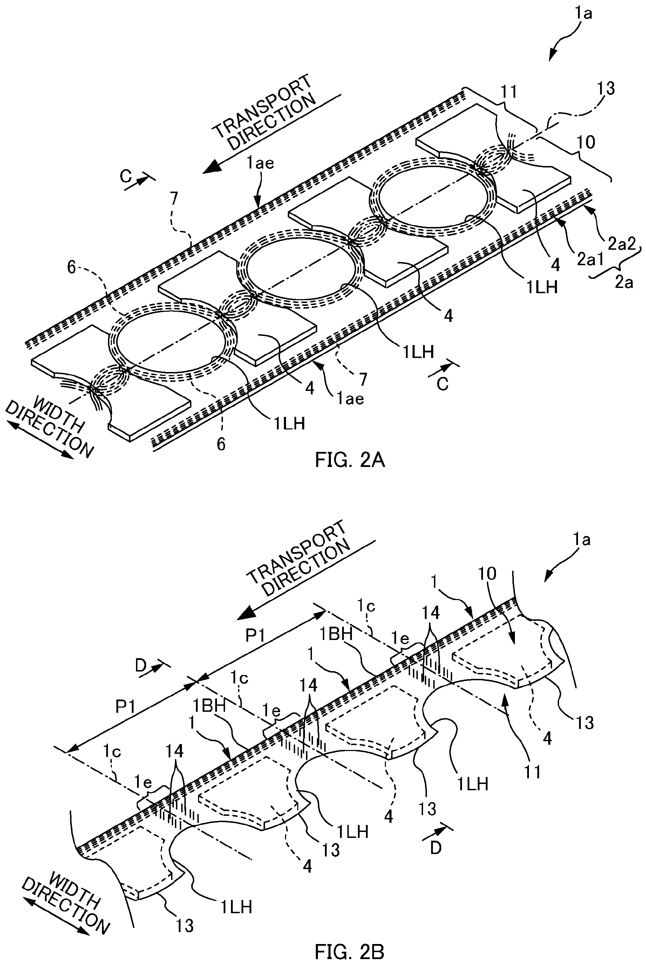

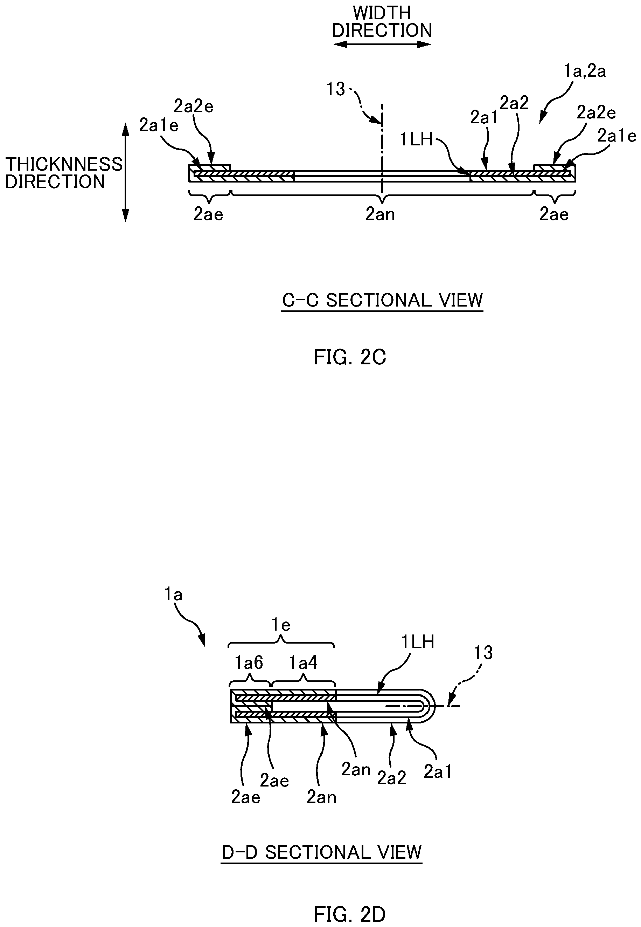

FIGS. 2A and 2B are schematic perspective views and illustrate how the substrate 1a of the diaper 1 is transported to the ultrasonic welding apparatus 20. FIG. 2A shows the substrate 1a of the diaper 1 which has not two-folded yet, and FIG. 2B shows the two-folded substrate 1a immediately before being transported to the ultrasonic welding apparatus 20. FIG. 2C is a cross-sectional view taken along line C-C of FIG. 2A. FIG. 2D is a cross-sectional view taken along line D-D of FIG. 2B. In both FIGS. 2C and 2D, an absorbent body 4, leg-circumference elastic members 6, and waist-circumference elastic members 7 are not shown.

The substrate 1a of the diaper 1 includes a continuous sheet 2a which continues in the transport direction. At the time point shown in FIG. 2A, absorbent main bodies 4, 4... are placed with spacing of a product pitch P1 in the transport direction and joined, with adhesive or the like, onto a surface of the continuous sheet 2a which is to face wearer's skin.

In the substrate 1a at this time, leg openings 1LH each are formed at a position between absorbent main bodies 4 and 4 which are immediately adjacent to each other in the transport direction. Leg-circumference elastic members 6, which provide stretchability to the leg openings 1LH, are attached along the leg opening 1LH. In addition, waist-circumference elastic members 7, which provide stretchability to the waist opening 1BH, are attached along end edge parts 1ae and 1ae which are to be the waist opening 1BH.

The continuous sheet 2a is configured with a sheet 2a having a two layer structure, for example (see FIG. 2C). That is, the continuous sheet 2a includes: a continuous sheet 2a1 (hereinafter referred to as an inner-layer sheet 2a1) which faces the skin side of a wearer to serve as an inner layer when the diaper 1 is worn; and a continuous sheet 2a2 (hereinafter referred to as an outer-layer sheet 2a2) which faces the non-skin side of a wearer to serve as an outer layer when it is worn. The inner-layer sheet 2a1 and the outer-layer sheet 2a2 are stacked in the thickness direction and are joined by means such as adhesion or welding.

In this example, the outer-layer sheet 2a2 is greater in the width direction than the inner-layer sheet 2a1. Thus, the outer-layer sheet 2a2 extends outwardly relative to both ends 2a1e and 2a1e in the width direction of the inner-layer sheet 2a1, in a state where the inner-layer sheet 2a1 is layered on the outer-layer sheet 2a2. Then, each of such outwardly extending parts 2a2e and 2a2e is turned back inwardly in the width direction such that the parts 2a2e and 2a2e cover the ends 2a1e and 2a1e in the width direction of the inner-layer sheet 2a1 as shown in FIG. 2C. Thus, each of end parts 2ae and 2ae in the width direction of the continuous sheet 2a has a three-layer structure in which three sheets are stacked. To be precise, a part 2an which is the continuous sheet 2a excluding the end parts 2ae and 2ae has a two-layer structure.

It is to be noted that an example of raw material of these inner-layer and outer-layer sheets 2a1 and 2a2 includes nonwoven fabric, woven fabric or a film made of a thermally weldable material such as thermoplastic resin. But, it is not limited thereto as long as it is a material capable of being ultrasonically welded, that is, a material capable of being melted and joined by heat which is generated by friction heating due to application of ultrasonic energy.

The absorbent main body 4 is a component which absorbs excretion liquid, and the main body thereof is a body formed by shaping liquid absorbent fiber (e.g. pulp fiber) or liquid-absorbent particulate matter (e.g. super absorbent polymer) into a predetermined body (e. g. a substantially hourglass shape). Such a shaped body is covered with a liquid-permeable cover sheet (not shown) such as tissue paper or nonwoven fabric. In addition, the shaped body is covered with a liquid-impermeable leak-proof sheet from the non-skin side.

Immediately before being fed into the ultrasonic welding apparatus 20, such a substrate 1a shown in FIG. 2A is two-folded at a crotch part 13, which is the substantially center of the substrate 1a in its width direction. Then, the substrate 1a which has been two-folded as shown in FIG. 2B is transported to the ultrasonic welding apparatus 20. In other words, a part corresponding to the front piece 10 of a diaper 1 and a part corresponding to the back piece 11 are layered in the up-down direction and are transported to the ultrasonic welding apparatus 20.

However, at this time, the substrate 1a of the diaper 1 is in a state in which the part corresponding to the front piece 10 and the part corresponding to the back piece 11 are layered but have not been joined yet. Accordingly, the ultrasonic welding apparatus 20 forms the welded part 14 of the substrate 1a by welding the substrate 1a on its part 1e which corresponds to waist-circumferential side-end parts 1e of a diaper 1. Consequently, the front piece 10 and the back piece 11 of the substrate 1a are joined.

Here, the parts 1e which are to be welded, that is, the parts 1e each of which corresponds to waist-circumferential side-end parts 1e of a diaper 1 are placed in the substrate 1a at a product pitch P1 in the transport direction on both sides of each absorbent main body 4. Accordingly, the ultrasonic welding apparatus 20 forms welded parts 14 on both side parts 1e of each absorbent main body 4 in the substrate 1a, at the product pitch P1 in the transport direction. At this time, as shown in FIG. 2B, for each of to-be-welded parts 1e, at least a pair of welded parts 14 and 14 are formed being lined in the transport direction. Then, the substrate 1a in which these welded parts 14 are formed is transported to a downstream process, and in the downstream process, the substrate 1a is subsequently divided at a part 1c, which is between the pair of welded parts 14 and 14. Thereby, a diaper 1 having the waist opening 1BH and the leg openings 1LH is produced.

Incidentally, in a state where the substrate 1a has not been two-folded yet, which is a state of FIG. 2A, the continuous sheet 2a of the substrate 1a includes the three-sheet stacked part 2ae and the two-sheet stacked part 2an, as has been described with reference to FIG. 2C. Then, when the substrate 1a is two-folded as shown in FIG. 2B, the three-sheet stacked parts 2ae and 2ae are stacked on each other as well as the two-sheet stacked parts 2an and 2an are stacked on each other, as shown in FIG. 2D. As a result, the two-folded substrate 1a is in a state of including six-sheet stacked part 1a6 (corresponding to a first part) and a four-sheet stacked part 1a4 (corresponding to a second part). Therefore, the to-be-welded part 1e in the abovementioned substrate 1a also includes a six-sheet stacked part 1a6 and the four-sheet stacked part 1a4, and thus the ultrasonic welding apparatus 20 performs welding processes to the parts 1a4 and 1a6 having the numbers of stacked sheets 2a1, 2a2 ... different from each other, respectively, as mentioned above. This is associated with a characteristic structure of the invention, which will be described later.

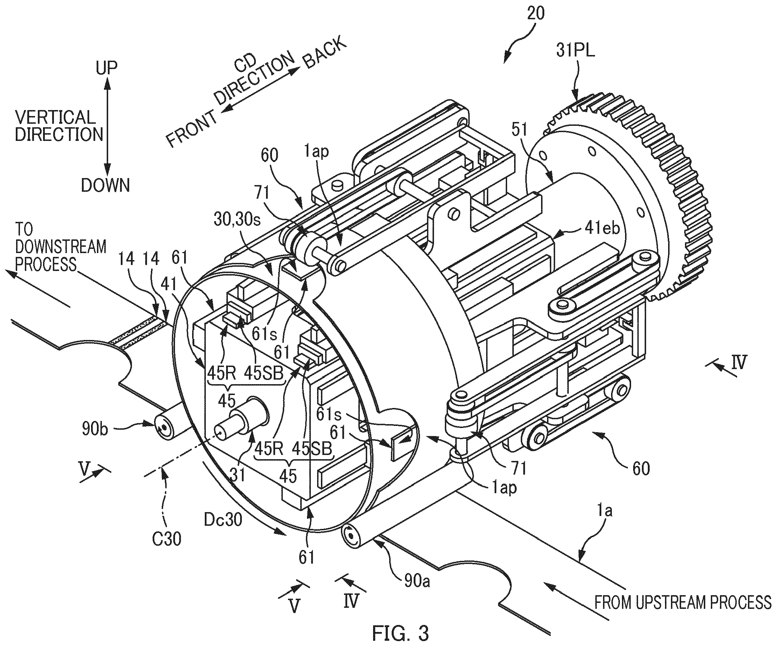

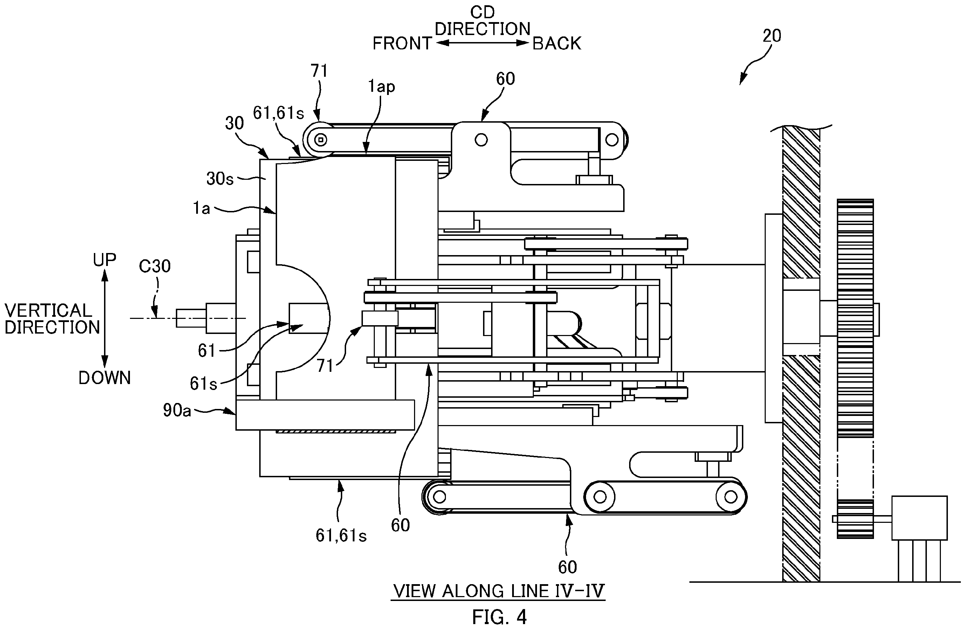

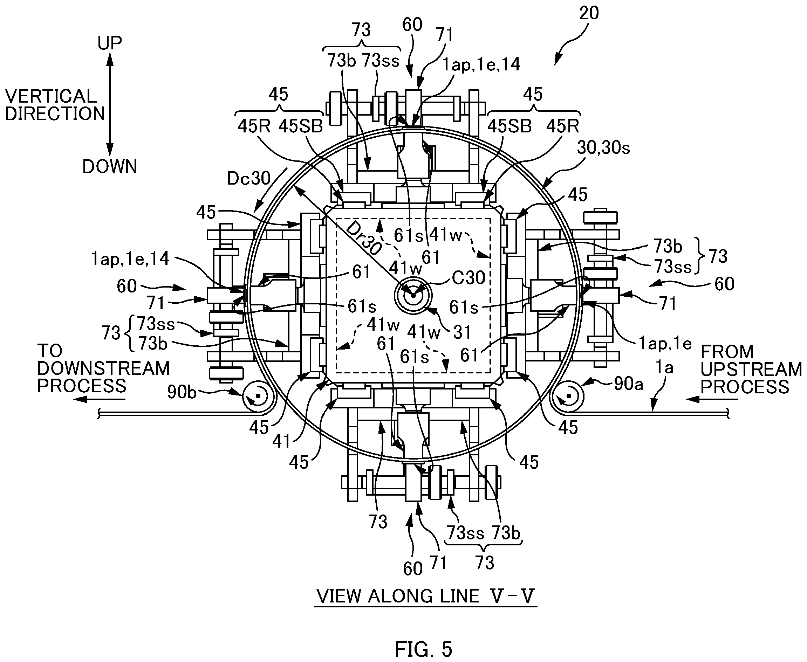

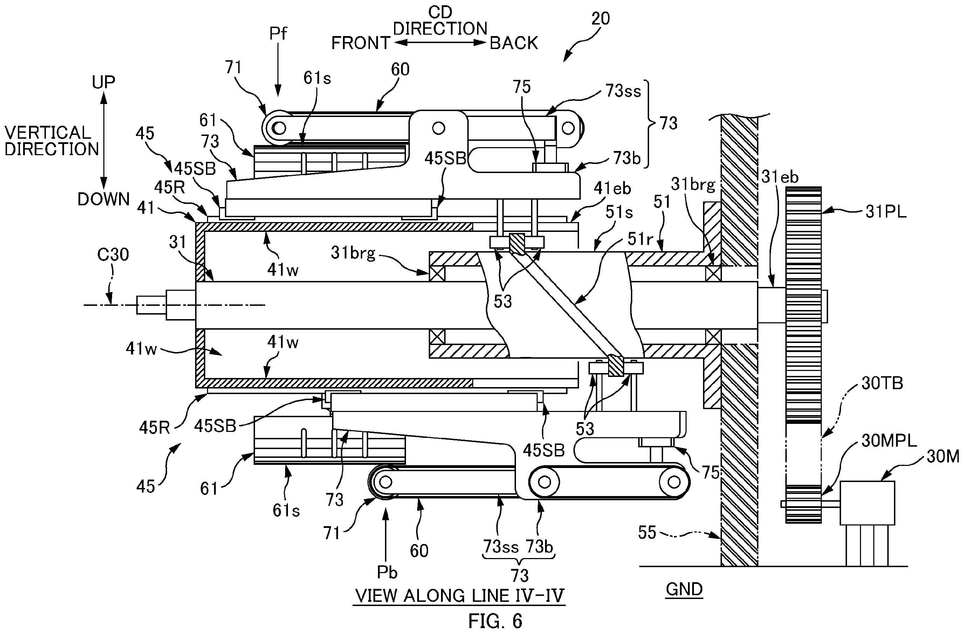

FIG. 3 is a schematic perspective view of the ultrasonic welding apparatus 20 as viewed obliquely from front above. FIG. 4 is a schematic side view along arrows IV-IV in FIG. 3, and FIG. 5 is a schematic front view along arrows V-V in FIG. 3. FIG. 6 is a schematic side view with the substrate 1a and a rotating drum 30 being removed from FIG. 4, and some components such as a column 41 being illustrated in a cut-away view.

In the description below, a direction orthogonal to the transport direction of the substrate 1a in the production line is referred to as a "CD direction". In this example, the CD direction is in the horizontal direction. And, a substrate 1a is being transported along a continuing direction in which the substrate 1a continues, and the width direction of the substrate 1a is designed to be parallel to the abovementioned CD direction. The thickness direction of the substrate 1a is orthogonal to both of the continuing direction and the width direction of the substrate 1a.

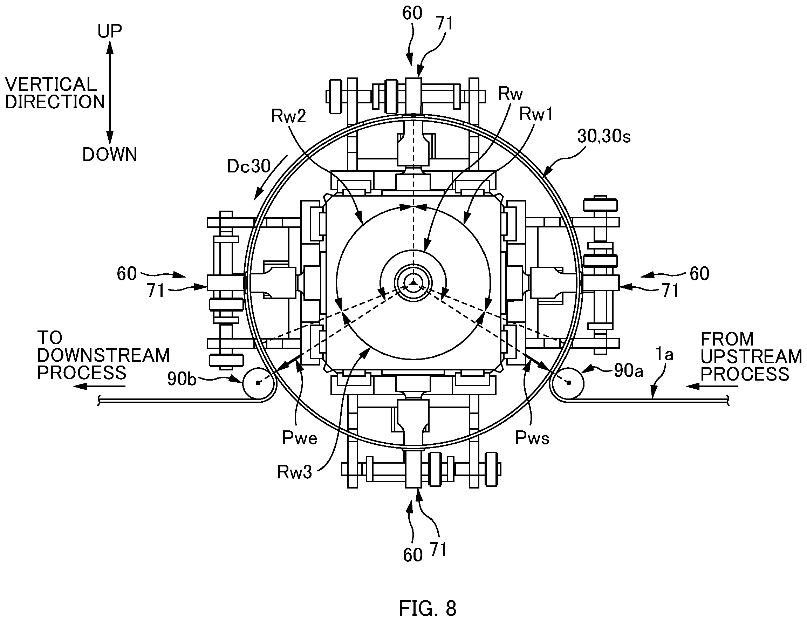

As shown in FIGS. 3 and 5, the ultrasonic welding apparatus 20 includes: the rotating drum 30 (corresponding to the rotating member); a plurality (four in this example) of ultrasonic processing units 60, 60...; and a pair of guide rolls 90a and 90b. The rotating drum 30 having a substantially cylindrical shape rotates in one direction about a central axis C30 which is along the CD direction. The ultrasonic processing units 60, 60... rotate about the central axis C30 together with the rotating drum 30. Concerning the substrate 1a which is being transported from an upstream process, the pair of guide rolls 90a and 90b transports the substrate 1a to the downstream process while winding the substrate 1a on the outer circumferential face 30s of the rotating drum 30 through a predetermined range Rw in the rotation direction Dc30 (FIG. 8).

The rotating drum 30 is driven and rotated at substantially the same circumferential speed value (m/min) as the transport speed value (m/min) of the substrate 1a which is being transported from the upstream process. Accordingly, the substrate 1a is being transported along the outer circumferential face 30s of the rotating drum 30 while the substrate 1a being wound on the outer circumferential face 30s, substantially without relative slippage to the substrate 1a. Then, after the substrate 1a has moved out of the predetermined range Rw, the substrate 1a is separated away from the outer circumferential face 30s and is transported to the downstream process.

In the description below, for the purpose of explanation, it is assumed that the circumferential speed value (m/min) of the rotating drum 30 is constant. That is, in an actual production line, the circumferential speed value (m/min) of the rotating drum 30 may fluctuate. For example, when starting or stopping the production line, or when being in a sudden trouble, the rotating drum 30 rotates at a circumferential speed value which is different from a constant circumferential speed value (m/min), which is a steady speed value. But, most of the operating time for producing the diapers 1, the rotating drum 30 rotates at a constant circumferential speed value (m/min), which is the abovementioned steady speed value. On the other hand, the rotating drum 30 is rotating at an unsteady speed value, for a very short time like when starting the production line. Since the description based on the foregoing assumption will not disturb understanding the concept of the invention, the description is made below based on the foregoing assumption.

As shown in FIGS. 3 and 5, the ultrasonic processing units 60, 60... are provided at every predetermined angle (e.g. 90°) in the rotation direction Dc30 of the rotating drum 30. Each ultrasonic processing unit 60 includes a horn 61 and a roller-like anvil 71 (corresponding to the roller member). The horn 61 vibrates ultrasonically and is arranged on the outer circumferential face 30s of the rotating drum 30 not to move relative to the outer circumferential face 30s. The anvil 71 is arranged outside with respect to the horn 61 in the rotation-radius direction Dr30 of the rotating drum 30 so that the substrate 1a is sandwiched between the anvil 71 and the horn 61.

Here, the horn 61 is in a rail-like shape extending in the CD direction, and roller-like anvil 71 (hereinafter referred to as an anvil roller 71) is capable of rolling along the CD direction on such a rail-like horn 61 (corresponding to the rail-like member). And, the anvil roller 71 is capable of reciprocating in the CD direction with respect to a part 1ap of the substrate 1a which is placed on the horn 61. Accordingly, during the reciprocation, ultrasonic energy is selectively applied from the horn 61 to a part 1ap of the substrate 1a which is sandwiched between the anvil roller 71 and the horn 61. Consequently, a welded part 14 is formed in the part 1ap of the substrate 1a.

The configuration of the ultrasonic welding apparatus 20 will be described in detail below.

As shown in FIG. 3, the main body of the rotating drum 30 is a cylinder, and its cross section in which its normal direction is the CD direction is in a circular shape, for example. At the circle center, which is the center of this cross section, a shaft member 31 is provided on the abovementioned central axis C30 in a concentric and integrated manner. In addition, the shaft member 31 is supported rotatably by the bearing member 31brg shown in FIG. 6 while the axial direction of the shaft member 31 being oriented in the CD direction. Accordingly, the rotating drum 30 is capable of rotating about the abovementioned central axis C30.

The rotating drum 30 is provided with rotational force by the servomotor 30M, which is a driving source, through a suitable rotational-force transmission mechanism. Accordingly, the rotating drum 30 is driven and rotated in one direction.

For example, in the example of FIG. 6, a so-called belt-type transmission device is used as the rotational-force transmission mechanism. That is, in the belt-type transmission device, an endless timing belt 30TB is wound around a pulley 31PL and a pulley 30MPL; the pulley 31PL is provided on the one end section 31eb of the shaft member 31 in a concentric and integrated manner, and the pulley 30MPL is provided on the driving rotational shaft of the servomotor 30M in a concentric and integrated manner. Thereby, driving rotational force generated by the servomotor 30M is transmitted to the shaft member 31, which serves as the central axis C30 of the rotating drum 30. Accordingly, the rotating drum 30 is driven and rotated by the servomotor 30M. However, rotational-force transmission mechanism is not limited thereto. For example, it is acceptable that the abovementioned pulleys 31PL and 30MPL are respectively replaced with gears to configure the rotational-force transmission mechanism with a group of gears. In this example, though the cross section of the rotating drum 30 is in a circular shape, it is not limited thereto. For example, the shape of the cross section may be a polygon, for example, a regular polygon having more corners than the number of the ultrasonic processing units 60.

As shown in FIG. 5, a plurality (e.g. four) of the ultrasonic processing units 60, 60... are provided at every predetermined angle in the rotation direction Dc30 of the rotating drum 30. The predetermined angle is set to an angle in which the length in the rotation direction Dc30 on the outer circumferential face 30s of the rotating drum 30 is substantially equal to a length corresponding to a single diaper. In the example of FIG. 5, the predetermined angle is set to 90°. Accordingly, the number of the ultrasonic processing units 60, 60... are provided is four. The servomotor 30M as a driving source is controlled by a control section (not shown) such as a computer, a programmable logic controller (PLC) or the like, and such control is performed so that the rotating drum 30 rotates by the predetermined angle as the substrate 1a is fed from the upstream process by a length corresponding to a single diaper. Accordingly, each to-be-welded part 1e of the substrate 1a is correlated to one of the ultrasonic processing units 60 and the ultrasonic welding process is performed. Such a rotational movement is realized, for example, by controlling the position of the abovementioned servomotor 30M based on synchronization signals.

The synchronization signals are outputted from a rotation detection sensor (not shown; e.g. a rotary encoder), which measures the transport amount of the substrate 1a in, for example, an apparatus which serves as a reference in the production line (e.g. a rotary die cutter apparatus which forms the leg opening 1LH of FIG. 2A by stamping). Such a synchronization signal is a rotational angle signal obtained by assigning each of the rotation angle values between 0° and 360° in proportion to the transport amount, with the transport amount corresponding to a single product diaper (substantially equal to the foregoing product pitch P1) being used as a unit transport amount, for example. Each time the transportation is performed by an amount of the diaper, the rotational angle value between 0° and 360° is outputted repeatedly and periodically. However, the synchronization signal is not limited to the rotational angle signal. For example, as the synchronization signal, a digital signal may be used which is obtained by assigning each of digital values of 0-8191 in proportion to the transport amount, to the above unit transport amount.

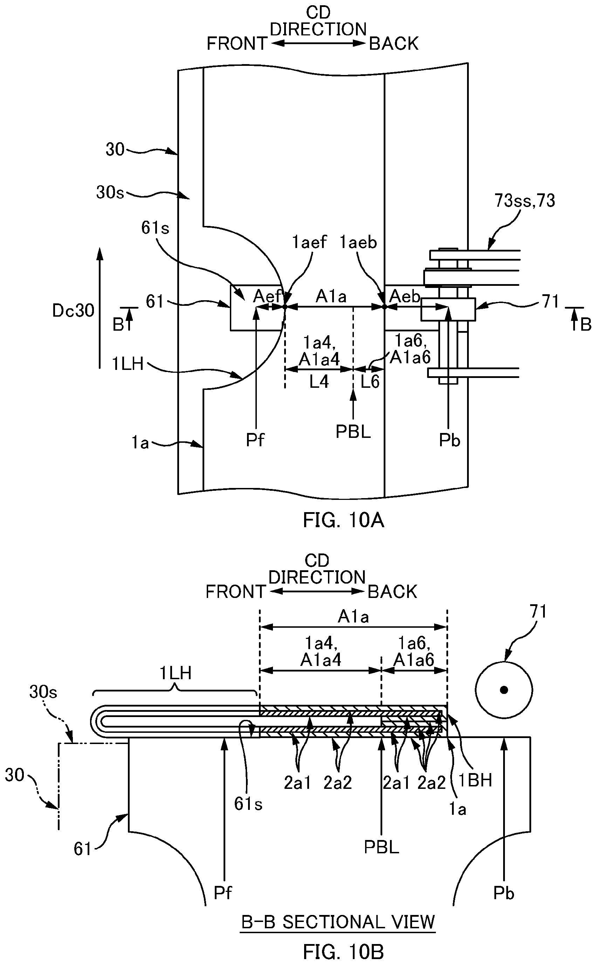

As illustrated in FIGS. 3 and 5, each ultrasonic processing unit 60 includes the rail-like horn 61 and the anvil roller 71. The horn 61 extends along the CD direction and is fixed to the column 41 (to be described later) so as to become immovable relative to the outer circumferential face 30s of the rotating drum 30. The anvil roller 71 is provided so as to reciprocate in the CD direction while rolling on the horn 61. As shown in FIGS. 3, 5 and 6, the horn 61 includes a substantially flat surface 61s facing outwards the rotation-radius direction Dr30 of the rotating drum 30, and the anvil roller 71 rolls on the substantially flat surface 61s. The substantially flat surface 61s serves as the oscillating surface 61s that vibrates ultrasonically. The oscillating surface 61s is fixed to the outer circumferential face 30s of the rotating drum 30 in a state in which the surface 61s coincides with the outer circumferential face 30s, or in which the surface 61s slightly protrudes outwards in the rotation-radius direction Dr30. The length of the oscillating surface 61s in the CD direction is designed to be a dimension in which the oscillating surface 61s extends beyond both sides of the substrate 1a in the CD direction, the substrate 1a being wound around the outer circumferential face 30s of the rotating drum 30 (e.g. see FIG. 10). Accordingly, based on reciprocation of the anvil roller 71 in the CD direction, the welded part 14 can be formed through the entire length of the substrate 1a in the CD direction.

The horn 61 is connected to an oscillator via a booster and a converter (all of them are not shown). The oscillator has an electric circuit, and the electric circuit generates electric signals having a certain frequency from 20 kHz to 35 kHz when power is supplied from a suitable power supply. The converter converts the electric signals having the certain frequency which has been sent from the oscillator, into mechanical vibration having the same frequency, by means such as a piezo element. The booster amplifies the mechanical vibration sent from the converter, and transmits it to the horn 61. Accordingly, the oscillating surface 61s of the horn 61 vibrates ultrasonically in the direction normal to the surface 61s.

Here, ultrasonic energy is applied to the substrate 1a using ultrasonic vibration of the oscillating surface 61s of the horn 61. If the frequency of the ultrasonic vibration is constant, the amount (J) of the ultrasonic energy can be changed by any of the following methods: changing the amplitude of the ultrasonic vibration; or changing the magnitude (N) of force at which the substrate 1a is sandwiched between (by) the oscillating surface 61s and the anvil roller 71 (hereinafter referred to as the sandwiching force). For example, if the magnitude (N) of sandwiching force is constant, the resistance to the vibration increases/decreases in conjunction with increase/decrease in the amplitude. Consequently, the power consumption of the oscillator increases/decreases. Since most of the power consumption is applied to the substrate 1a as ultrasonic energy, ultrasonic energy that is applied to the substrate 1a increases/decreases in conjunction with increase/decrease in the amplitude. On the other hand, if the amplitude is constant, the resistance to the vibration increases/decreases in conjunction with increase/decrease in the magnitude (N) of sandwiching force. Consequently, the power consumption of the oscillator also increases/decreases, and most of the power consumption is applied to the substrate 1a as ultrasonic energy. That is, ultrasonic energy that is applied to the substrate 1a increases/decreases in conjunction with increase/decrease in the magnitude (N) of sandwiching force.

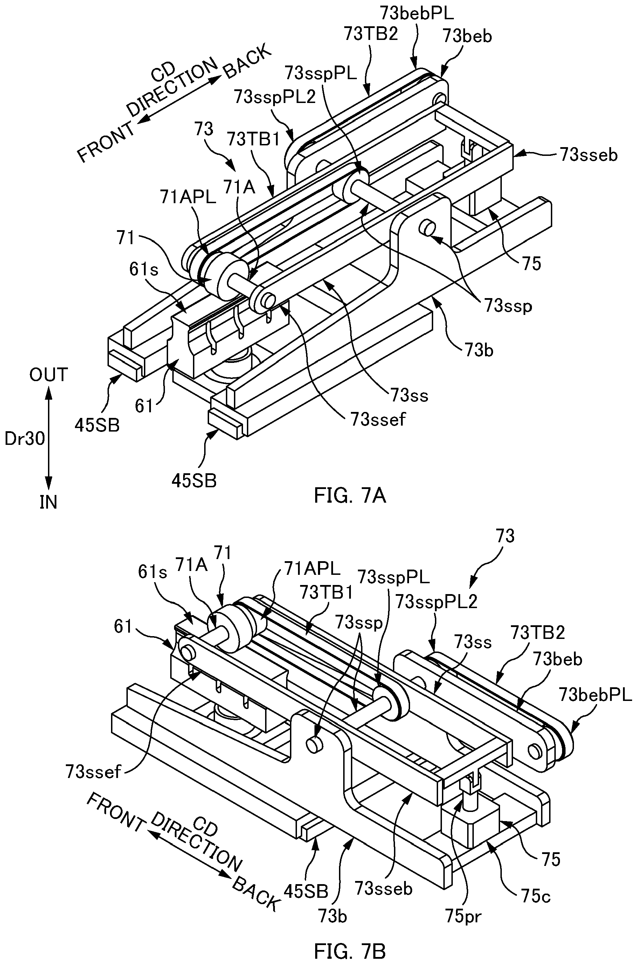

In the former case, changing the amplitude can be made by an oscillator. That is, the abovementioned oscillator is capable of changing the amplitude of the ultrasonic vibration to any value, based on control signals transmitted from the ultrasonic-energy regulating unit (not shown), the ultrasonic-energy regulating unit being configured with a computer, a PLC, or the like. And, in the latter case, changing the magnitude (N) of sandwiching force can be made by an air cylinder 75 (to be described later; see FIGS. 7A and 7B) provided in the anvil roller 71. This will be described later. In this example, the oscillator is capable of regulating the amplitude of the ultrasonic vibration (the distance from the balanced position to the maximum displacement) to any value between 0-30 µm. However, the regulatable range of the amplitude is not limited thereto.

On the other hand, as mentioned with reference to FIGS. 5 and 6, the anvil roller 71 faces the oscillating surface 61s of the horn 61, and is arranged outside with respect to the oscillating surface 61s in the rotation-radius direction Dr30 of the rotating drum 30. The anvil roller 71 is provided so as to reciprocate in the CD direction while rolling on the oscillating surface 61s. For example, the reciprocation of the anvil roller 71 is implemented as follows.

As shown in FIGS. 3, 5 and 6, inside the rotating drum 30, a column 41 having a polygonal tubular shape is provided coaxially with the foregoing shaft member 31, which serves as the central axis C30 of the rotating drum 30. Most part of the column 41 is accommodated inside the rotating drum 30, and the one end section 41eb of the column 41 in the CD direction protrudes beyond the rotating drum 30. The column 41 is connected to and integrated with the shaft member 31 of the rotating drum 30 using a connecting member (not shown). Accordingly, the column 41 rotates about the central axis C30 together with the rotating drum 30. In the description below, concerning the CD direction, a direction toward which the column 41 protrudes beyond the rotating drum 30 is called as "back", and the opposite direction is called as "front".

As shown in FIG. 5, a cross section of the column 41 (a cross section in which its normal direction is the CD direction) is a regular polygon having the same number of corners as the number of the ultrasonic processing units 60, 60..., for example. Accordingly, the column 41 is a tubular body having the same number of wall sections 41w, 41w... as the number of the ultrasonic processing units 60, 60.... In the example of FIG. 5, the column 41 includes four ultrasonic processing units 60, 60..., and therefore the column 41 has a square cross section. In other words, the column 41 is a square tubular body having four wall sections 41w, 41w.... The wall sections 41w respectively correspond to the ultrasonic processing units 60 one to one. That is, each wall section 41w includes a linear guide 45 for reciprocating the anvil roller 71 of the ultrasonic processing unit 60 in the CD direction. As shown in FIG. 6, the linear guide 45 includes: a rail 45R which is fixed to the wall section 41w and extends in the CD direction; and sliding blocks 45SB and 45SB which are engaged with the rail 45R so as to be able to slide towards both sides in the CD direction. A support unit 73, which supports the anvil roller 71, is fixed to the sliding blocks 45SB and 45SB.

FIGS. 7A and 7B are diagrams illustrating the support unit 73. FIG. 7A is a schematic perspective view of the support unit 73 as viewed obliquely from front above and outside in the rotation-radius direction Dr30, and FIG. 7B is a schematic perspective view of the support unit 73 as viewed obliquely from back and outside in the rotation-radius direction Dr30.

The support unit 73 includes: a base section 73b fixed to the sliding blocks 45SB, 45SB...; and a seesaw-like member 73ss which is supported by the base section 73b so as to be capable of oscillating and extends in the CD direction. Here, the seesaw-like member 73ss is supported by the base section 73b with a support shaft 73ssp so as to be capable of oscillating, the support shaft 73ssp being provided at substantially the center in the CD direction. That is, the front-end part 73ssef and the back-end part 73sseb of the seesaw-like member 73ss are each capable of oscillating in the rotation-radius direction Dr30 of the rotating drum 30. More specifically, the front-end part 73ssef and the back-end part 73sseb move in nearly opposite directions to each other. In the front-end part 73ssef, the abovementioned anvil roller 71 is supported rotatably. On the other hand, a double-acting air cylinder 75 is provided in the back-end part 73sseb as a driving source to drive the seesaw-like member 73ss to oscillate, for example.

As shown in FIG. 7B, the air cylinder 75 includes: a cylinder section 75c; a piston (not shown) which is capable of sliding inside the cylinder section 75c and which partitions the cylinder section 75c to form two pressure chambers; and a piston rod 75pr which is capable of coming out of and in the cylinder section 75c and which is integrated with the piston. The tip end of the piston rod 75pr is connected to the back-end part 73sseb of the seesaw-like member 73ss, and the cylinder section 75c is fixed to the base section 73b. Accordingly, by controlling the pressure (MPa) of compressed air being supplied to each of two pressure chambers, it is possible to press the anvil roller 71 against the oscillating surface 61s of the horn 61 and to separate the anvil roller 71 from the oscillating surface 61s, through the movement of the piston rod 75pr.

For example, if one pressure chamber is opened to the atmosphere and compressed air is supplied to the other pressure chamber, the substrate 1a can be sandwiched between the anvil roller 71 and the oscillating surface 61s of the horn 61. In addition, if the supply pressure (MPa) of the compressed air changes, it is possible to regulate the magnitude (N) of the sandwiching force for the substrate 1a. It should be noted that a mechanism for regulating the supply pressure (MPa) of compressed air which is supplied to the air cylinder 75 is exemplified by a configuration including pressure-regulator valves (not shown) and directional control valves (e.g. solenoid valves; not shown), the pressure-regulator valves being respectively provided in channels through which compressed air is supplied to the pressure chambers, the directional control valves respectively switching between connection and disconnection of the supply channels to a compressed-air source (not shown). However, it is not limited thereto.

On the other hand, rotational movement of the column 41 generates driving force for reciprocating the support unit 73 of the anvil roller 71 in the CD direction. That is, the column 41 rotates in the rotation direction Dc30 in an integrated manner with the rotating drum 30, and the ultrasonic welding apparatus 20 has a cam mechanism which drives the support units 73 by converting the rotational movement into reciprocation in the CD direction and transmitting it to the support units 73.

As shown in FIG. 6, such a cam mechanism has, for example, a cylindrical member 51 that is inserted into the column 41 in a coaxial manner with the column 41, and the cylindrical member 51 is fixed to a suitable support member 55 on the ground GND side so as to be unable to rotate. A ribbed cam 51r is provided on the outer circumferential face 51s of the cylindrical member 51, and a pair of cam followers 53 and 53 is provided in the base section 73b of the support unit 73. The abovementioned ribbed cam 51r is sandwiched between the pair of cam followers 53 and 53 in the front-back direction and are engaged with the cam followers 53 and 53. The ribbed cam 51r is provided in an endless manner continuously in the rotation direction Dc30 of the rotating drum 30. Further, the position of the cam 51r in the CD direction changes according to the position of the cam 51r in the rotation direction Dc30, and a cam curve is therefore set. The setting of the cam curve determines the reciprocation of the support unit 73.

For example, in this example, under the condition that the circumferential speed value (m/min) of the rotating drum 30 is constant, the abovementioned cam curve is set so that the support unit 73 moves at the same constant travel speed value (m/min), on both of the forward path and the return path of reciprocation of the support unit 73.

More specifically, as shown in a schematic front view of FIG. 8, a first angular range Rw1 and a second angular range Rw2 are set, in such a manner as to have the same magnitude and not to overlap each other, within a winding angular range Rw in which the substrate 1a is wound around the rotating drum 30. Here, a forward movement, a movement in the forward path, is assigned to a part of the cam curve which corresponds to the first angular range Rw1. For this purpose, that part has a shape in which the position of the ribbed cam 51r in the CD direction shifts forward in proportion to the change in the position of the ribbed cam 51r in the rotation direction Dc30. Accordingly, when the support unit 73 passes through the first angular range Rw1, the support unit 73 and the accompanying anvil roller 71 move at a constant travel speed value (m/min) from a backward limit Pb to a forward limit Pf, the backward limit Pb being provided at the back in the CD direction (FIG. 6), the forward limit Pf being provided at the front (FIG. 6). Further, a backward movement, a movement in the return path, is assigned to a part of the cam curve which corresponds to the second angular range Rw2. For this purpose, that part has a shape in which the position of the ribbed cam 51r in the CD direction shifts backward in proportion to the change in the position of the ribbed cam 51r in the rotation direction Dc30. Accordingly, when the support unit 73 passes through the second angular range Rw2, the support unit 73 and the accompanying anvil roller 71 move from the forward limit Pf to the backward limit Pb at the same travel speed value (m/min) as in the forward path.

In the example of FIG. 8, the first angular range Rw1 and the second angular range Rw2 are provided to be adjacent to each other. Accordingly, the anvil roller 71 which has reached the forward limit Pf as a result of the forward movement promptly turns and performs the backward movement. However, it is not limited thereto. For example, the anvil roller 71 may performs the backward movement after the anvil roller 71 stops for some period of time at the forward limit Pf.

In a state in which the anvil roller 71 is positioned at the backward limit Pb, the anvil roller 71 has completely finished crossing the substrate 1a, and is not in contact with the substrate 1a (see FIGS. 10A and 10B, for example). The abovementioned cam curve is set so that the anvil roller 71 stays at the backward limit Pb within an angular range Rw3 in the rotation direction Dc30, the angular range Rw3 being a range except for the first and second angular ranges Rw1 and Rw2. Thus, the anvil roller 71 is positioned at the backward limit Pb, at both of a position of the initial end of the winding angular range Rw of the substrate 1a serving as a winding start position Pws and a position of the final end of the winding angular range Rw serving as a winding end position Pwe. This allows the anvil roller 71 not to interrupt an operation of winding the substrate 1a onto the outer circumferential face 30s of the rotating drum 30 and an operation of releasing the substrate 1a from the outer circumferential face 30s.

Such an anvil roller 71 is configured to rotate according to its reciprocation in the CD direction. That is, during the forward movement, the roller 71 is rotating forward at the circumferential speed value (m/min) substantially the same as the travel speed value (m/min) so as to roll forward. And, during the backward movement, the roller 71 is rotating backward at the circumferential speed value (m/min) substantially the same as the travel speed value (m/min) so as to roll backward. Accordingly, the anvil roller 71 is rolling on the substrate 1a and moving in the CD direction, substantially without relative slippage with respect to the substrate 1a.

Such rotational movements of the anvil roller 71 may be made without its driving source. For example, as shown in FIGS. 7A and 7B, since the anvil roller 71 is supported by the support units 73 through suitable bearing member (not shown), the anvil roller 71 is capable of rotating at an extremely small rotational resistance. And, the foregoing air cylinder 75 presses the anvil roller 71 against the oscillating surface 61s of the horn 61 via the substrate 1a. Accordingly, in accordance with the reciprocation of the anvil roller 71, the anvil roller 71 obtains rotational force from the substrate 1a and rotates. Then, the anvil roller 71 is rotating as a follower.

However, the foregoing rotation of the anvil roller 71 as a follower leads to a problem of reliability of the rotational movement. Then, in the example of FIGS. 7A and 7B, for a purpose of ensuring the rotation of the anvil roller 71, there is provided a movement converting mechanism which converts the reciprocation of the support unit 73 into rotational movement and transmits it to the anvil roller 71. Such a movement converting mechanism is a so-called belt-type transmission mechanism.

That is, a pulley 71APL is fixed to a shaft section 71A which is integrated and concentric with the anvil roller 71, and another pulley 73sspPL is rotatably supported by the support shaft 73ssp of the seesaw-like member 73ss. An endless timing belt 73TB1 is wound around these pulleys 71APL and 73sspPL. To the latter pulley 73sspPL, a pulley 73sspPL2 is integrated and fixed in a concentric manner. In addition, another pulley 73bebPL is rotatably supported in a back-end part 73beb of the base section 73b of the support unit 73. Another endless timing belt 73TB2 is wound around these pulleys 73sspPL2 and 73bebPL. In addition, a part of the timing belt 73TB2 is connected to the column 41 through a connecting member.

Accordingly, when the support unit 73 is moving in the CD direction relative to the column 41, the anvil roller 71 is rotating by the amount of this movement. That is, when the support unit 73 moves forward, the anvil roller 71 rotates in the forward direction by the same amount as the amount of this forward movement. On the other hand, when the support unit 73 moves backward, the anvil roller 71 rotates in the backward direction by the same amount as the amount of this backward movement. This allows the anvil roller 71 to reliably roll on the substrate 1a during its reciprocation in the CD direction, substantially without relative slippage with respect to the substrate 1a.

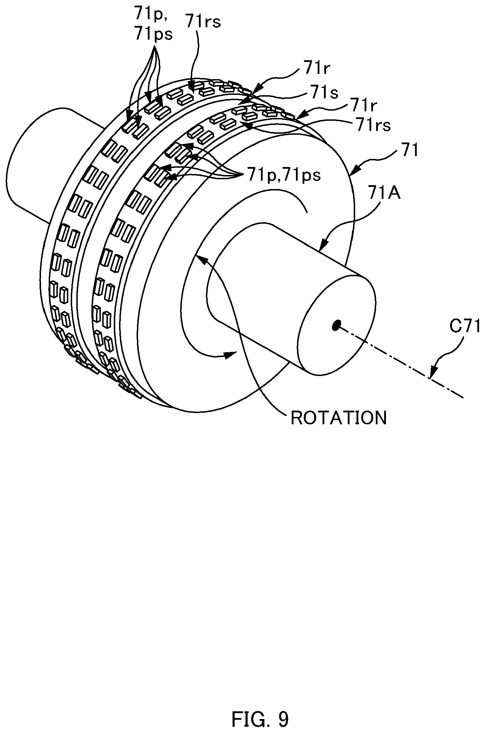

FIG. 9 is a schematic perspective view of the anvil roller 71. On the circumferential face 71s of the anvil roller 71, a plurality of protrusions 71p, 71p... are provided to form the welded parts 14 in a pattern on the substrate 1a. Specifically speaking, on the circumferential face 71s of the anvil roller 71, two endless ribs 71r and 71r are provided side by side in a direction along the rotational axis C71 of the anvil roller 71, and the ribs 71r and 71r extend through the entire length in its circumferential direction. On the top surface 71rs of each rib 71r, a plurality of island-shaped protrusions 71p, 71p... are provided in a pattern having certain regularity. And, the top surfaces 71rs of the ribs 71r form low-compressed portions in the substrate 1a, and the top surfaces 71ps, 71ps... of the plurality of protrusions 71p, 71p... form high-compressed portions at a compression rate greater than that in the low-compressed portions. Accordingly, the welded parts 14 are formed in a pattern including a plurality of high-compressed portions.

FIG. 10A is a schematic diagram illustrating a relative positional relationship between the horn 61 and the substrate 1a wound onto the outer circumferential face 30s of the rotating drum 30. FIGS. 10B is a cross-sectional diagram taken along line B-B of FIG. 10A.

Here, as shown in FIG. 10A, a section on the outer circumferential face 30s of the rotating drum 30 in which the anvil roller 71 crosses the substrate 1a along the CD direction is defined as a "crossing section A1a". Such a crossing section A1a, as shown in FIG. 10B, includes parts 1a6 and 1a4, in which the number of stacked sheets 2a1 and 2a2 configuring the substrate 1a different from each other. That is, the six-sheet stacked part 1a6, in which two inner-layer sheets 2a1 and four outer-layer sheets 2a2 are stacked, exists in a back edge part in the CD direction of the substrate 1a. On the other hand, the four-sheet stacked part 1a4, in which two inner-layer sheets 2a1 and two outer-layer sheets 2a2 are stacked, exists in a part 1a4 which is a part except the above.

As such, when the substrate 1a includes a plurality of parts 1a4 and 1a6 in the CD direction, in which the number of stacked sheets are different from each other, the amount (J/m) of ultrasonic energy necessary for welding varies with the number of such stacked sheets. That is, when the four-sheet stacked part 1a4 and the six-sheet stacked part 1a6 exist in the CD direction, as in the abovementioned substrate 1a, the six-sheet stacked part 1a6 needs ultrasonic energy greater than the ultrasonic energy necessary for welding of the four-sheet stacked part 1a4. Thus, if the six-sheet stacked part 1a6 is welded with the amount (J/m) of ultrasonic energy for the four-sheet stacked part 1a4, shortage of the applied ultrasonic energy may cause a joint failure. To the contrary, if the four-sheet stacked part 1a4 is welded with the amount (J/m) of ultrasonic energy for the six-sheet stacked part 1a6, excessive application of ultrasonic energy may cause the part 1a4 to be melted resulting in a loss thereof.

Accordingly, in this ultrasonic welding apparatus 20, as shown in FIG. 10B, a section corresponding to the six-sheet stacked part 1a6 in the crossing section A1a is defined as a "six-sheet section A1a6" (corresponding to a first section), and a section corresponding to the four-sheet stacked part 1a4 in the crossing section A1a is defined as a "four-sheet section A1a4" (corresponding to a second section). In such a case, the amount (J/m) of ultrasonic energy applied per unit length in the CD direction when the anvil roller 71 passes through the six-sheet section A1a6 and the amount (J/m) of ultrasonic energy applied per unit length in the CD direction when the roller 71 passes through the four-sheet section A1a4 are made different from each other.

The details are as follows. First, in this example, the amount (J/m) of ultrasonic energy necessary for welding is applied to the substrate 1a on the forward path, and the amount (J/m) of ultrasonic energy enough to avoid welding is applied thereto on the return path. Accordingly, the ultrasonic welding process is performed principally on the forward path while the welding process is basically kept from being performed onto the substrate 1a on the return path. Then, on the forward path, when the roller 71 passes through the six-sheet section A1a6, ultrasonic energy is applied, with the amount (J/m) thereof per unit length in the CD direction being maintained at a first predetermined value (J/m), which is necessary for welding of six-sheets. Further, on the same forward path, when the roller 71 passes through the four-sheet section A1a4 after passing though the abovementioned six-sheet section A1a6, ultrasonic energy is applied, with the amount (J/m) thereof per unit length in the CD direction being maintained at a second predetermined value (J/m), which is necessary for welding of four-sheets. Such a second predetermined value (J/m) is to be set smaller than the abovementioned first predetermined value (J/m).

Thus, in the six-sheet section A1a6, the six-sheet stacked part 1a6 in the substrate 1a can be welded without a joint failure or the like, and also in the four-sheet section A1a4, the four-sheet stacked part 1a4 in the substrate 1a can be welded without a melting loss or the like.

As such, the ultrasonic-energy regulating unit controls the oscillator so as to make the amount (J/m) of applied ultrasonic energy different between the six-sheet section A1a6 and the four-sheet section A1a4. The details are as follows.

First, the ultrasonic-energy regulating unit is always monitoring the position of the anvil roller 71 in the CD direction during its reciprocation. Such monitoring is performed, for example, indirectly based on signals outputted from a rotary encoder (not shown), which serves as a rotation detection sensor that detects the rotational angle of the rotating drum 30. That is, as mentioned above, the correspondence relationship between the position of the rotating drum 30 in the rotation direction Dc30 and that in the CD direction is uniquely predetermined based on the cam curve of the ribbed cam 51r. As a result, if the position of the anvil roller 71 in the rotation direction Dc30 of the rotating drum 30 is determined, it is possible to recognize the current position of the anvil roller 71 in the CD direction. On the other hand, the position of the anvil roller 71 in the rotation direction Dc30 can be detected based on the rotational angle value (0° to 360°) indicated by a signal outputted from the encoder.

Thus, the memory of the regulating unit stores, for example, data on the rotational angle value corresponding to the boundary position PBL (FIGS. 10A and 10B) between the abovementioned six-sheet section A1a6 and the four-sheet section A1a4 or data on the rotational angle value corresponding to a position close to the boundary position PBL, on the forward path. That is, the memory stores, in advance, the data on the rotational angle value corresponding to the position immediately before crossing the boundary position PBL, the data on the rotational angle value corresponding to the position of crossing the boundary position PBL, or the data on the rotational angle value corresponding to the position immediately after crossing the position PBL.

When the regulating unit detects that the rotational angle value indicated by a signal outputted from the abovementioned encoder is equal to or exceeds the abovementioned rotational angle value in the memory, the amplitude is changed from the greater amplitude for six-sheets to the amplitude for the four-sheets which is smaller than the above greater amplitude, simultaneously with or after an elapse of a predetermined time period from the detection. Thereby, the amount (J/m) of ultrasonic energy suitable for six-sheets can be applied to the six-sheet section A1a6, while the amount (J/m) thereof suitable for four-sheets can be applied to the four-sheet section A1a4.

It should be noted that, in this example, the amplitude on the return path is changed to the amplitude further smaller than the abovementioned amplitude for four-sheets. As a result, the ultrasonic welding process is kept from being performed on the return path. Such a change to the amplitude for the return path is made, for example, immediately before the anvil roller 71 has finished crossing the crossing section A1a on the forward path; at the time when the roller has finished crossing the section; or immediately after the roller has finished crossing the section. And, such a change is made, similarly to the above, also based on both the data on the rotational angle value stored in advance in the memory of the ultrasonic-energy regulating unit and the output signal of the abovementioned encoder. That is, the abovementioned memory stores, in advance, the data on the rotational angle value corresponding to the position of the anvil roller 71 immediately before the anvil roller 71 finishes crossing the abovementioned crossing section A1a; the data on the rotational angle value corresponding to the position of the anvil roller 71 when the anvil roller 71 has finished crossing the crossing section A1a; the data on the rotational angle value corresponding to the position of the anvil roller 71 immediately after the anvil roller 71 has finished crossing the crossing section A1a; or the like. When the regulating unit detects that the rotational angle value indicated by a signal outputted from the encoder becomes equal to or exceeds such a rotational angle value in the memory, the amplitude is changed to the smaller amplitude for the return path, simultaneously with or after an elapse of a predetermined time period from such detection.

In the process in which the anvil roller 71 returns back from the return path to the forward path, the amplitude is returned back to the greater amplitude for six-sheets. Such a change in amplitude is made, for example, immediately before the anvil roller 71 has finished crossing the crossing section A1a; at the time when the roller has finished crossing the section; or immediately after the roller has finished crossing the section, on the return path. That is, the memory of the regulating unit stores, in advance, the data on the rotational angle value corresponding to the position of the anvil roller 71 immediately before the anvil roller 71 finishes crossing the abovementioned crossing section A1a; the data on the rotational angle value corresponding to the position of the anvil roller 71 when the anvil roller 71 finishes crossing the crossing section A1a; the data on the rotational angle value corresponding to the position of the anvil roller 71 immediately after the anvil roller 71 has finished crossing the crossing section A1a; or the like, on the return path. When the regulating unit detects that the rotational angle value indicated by a signal outputted from the encoder becomes equal to or exceeds such a rotational angle value in the memory, the amplitude is changed from the smaller amplitude for the return path to the greater amplitude for six-sheets, simultaneously with or after an elapse of a predetermined time period after such detection. Accordingly, the amplitude is returned back to the amplitude for six-sheets, through which the anvil roller 71 is to pass first on the forward path.

As a matter of course, the first predetermined value (J/m) corresponding to the abovementioned six-sheet section A1a6 and the second predetermined value (J/m) corresponding to the four sheet section A1a4 are respectively set at optimum values of ultrasonic energy to be applied to the sections A1a6 and A1a4. However, strictly speaking, optimum values of the amounts (J/m) of ultrasonic energy to be applied in the six-sheet section A1a6 and the four-sheet section A1a4 can vary with a configuration of the substrate 1a and the like. Thus, optimum values of the amplitudes determined based on the abovementioned first predetermined value (J/m) and the second predetermined value (J/m) can also vary with a configuration of the substrate 1a and the like. Therefore, optimum values of the amplitudes are acquired through actual experiments in which the ultrasonic welding process is actually performed to the substrate 1a using the ultrasonic processing apparatus 20. However, the following criteria with respect to such optimum values can be provided. That is, optimum values of the amplitudes in the six-sheet section A1a6 having the greater number of stacked sheets is set, for example, to any value within a range from 15 µm to 30 µm. In this example, the amplitude is set to 30 µm. On the other hand, the amplitude in the four-sheet section A1a4 having the smaller number of stacked sheets is set, for example, to any value within a range from 50% to 90% of the amplitude in the section A1a6 having the greater number thereof. Preferably, the amplitude is set to any value within a range from 70% to 80% thereof. In this example, the amplitude is set to 80% thereof. Such setting makes it possible to reliably restrain defects such as a joint failure in the six-sheet section A1a6 and a melting loss in the four-sheet section A1a4.

In this example, the magnitude (N) of the sandwiching force with which the substrate 1a is sandwiched between the oscillating surface 61s of the horn 61 and the anvil roller 71 is maintained at a constant value throughout the crossing section A1a on the forward path. As a result, such a change in the amplitude as described above can reliably make the amount (J/m) of ultrasonic energy applied in the four-sheet section A1a4, smaller than the amount (J/m) of ultrasonic energy applied in the six-sheet section A1a6. Such an optimal value of the magnitude (N) of the sandwiching force can be obtained through actual experiments, etc., similarly to the above.

Incidentally, on the return path, an operation of separating the anvil roller 71 from the substrate 1a using the foregoing air cylinder 75 is also possible. But, in the above example, the horn 61 and the anvil roller 71 cooperate with each other to sandwich the substrate 1a therebetween throughout the crossing section A1a even on the return path. That is, on both of the forward path and the return path, the horn 61 and the anvil roller 71 are in contact with the substrate 1a. In addition, the anvil roller 71, which is capable of being separated from the substrate 1a, is not separated from the substrate 1a. Accordingly, the positional relationship between the anvil roller 71 and the horn 61 in the crossing section A1a can be kept in a constant state. This makes it possible to stabilize the ultrasonic welding process. The separating operation needs to be performed in an infinitesimally short time period, i.e., a part of a time period of hundreds of milliseconds, which is difficult to be performed. In Specific, the number of welding processes to be generally performed by a single ultrasonic unit 60 during one minute is from dozens of times to hundreds of times. Thus, the reciprocation in the CD direction of the anvil roller 71 is to be performed in hundreds of milliseconds. In association therewith, the above separating operation is also to be performed in a part of the abovementioned hundreds of milliseconds, however, the implementation of the separating operation in such an infinitesimally short time period is difficult in practice. Thus, whether the ultrasonic welding apparatus 20 can be implemented could be doubtful. However, in this example, since the anvil roller 71 is not separated from the substrate 1a, the ultrasonic welding apparatus 20 can be realized without any problem.

Incidentally, in this example, the amount (J/m) of ultrasonic energy necessary for welding of the substrate 1a is applied in the six-sheet section A1a6 and the four-sheet section A1a4 on the forward path, whereas the amount (J/m) of ultrasonic energy necessary for welding is not applied in the four-sheet section A1a4 and the six-sheet section A1a6 on the return path. However, it is not limited thereto. That is, the corresponding amounts (J/m) of ultrasonic energy may be applied in the four-sheet section A1a4 and the six-sheet section A1a6, not only on the forward path but also on the return path.

Further, contrary to the above, the ultrasonic energy may be applied not on the forward path but on the return path. That is, the amount (J/m) of ultrasonic energy necessary for welding may be applied in the four-sheet section A1a4 and the six-sheet section A1a6 on the return path, without being applied in the six-sheet section A1a6 and the four-sheet section A1a4 on the forward path. On such a return path, when the anvil roller 71 passes through the four-sheet section A1a4, the amount (J/m) of ultrasonic energy necessary for four-sheet welding is applied. And, on the same return path, when the anvil roller 71 passes through the six-sheet section A1a6 after passing through the above four-sheet section A1a4, the amount (J/m) of ultrasonic energy necessary for six-sheet welding is applied. In this case, needless to say, the amount (J/m) of ultrasonic energy necessary for four-sheet welding is smaller than the amount (J/m) of ultrasonic energy necessary for six-sheet welding.

Further, according to circumstances, the amount (J/m) of ultrasonic energy necessary for welding is applied in the six-sheet section A1a6 on the forward path, whereas the amount (J/m) of ultrasonic energy necessary for welding is not applied in the four-sheet section A1a4 on the same forward path. Instead, on the return path, the amount (J/m) of ultrasonic energy necessary for welding may be applied in the four-sheet section A1a4.

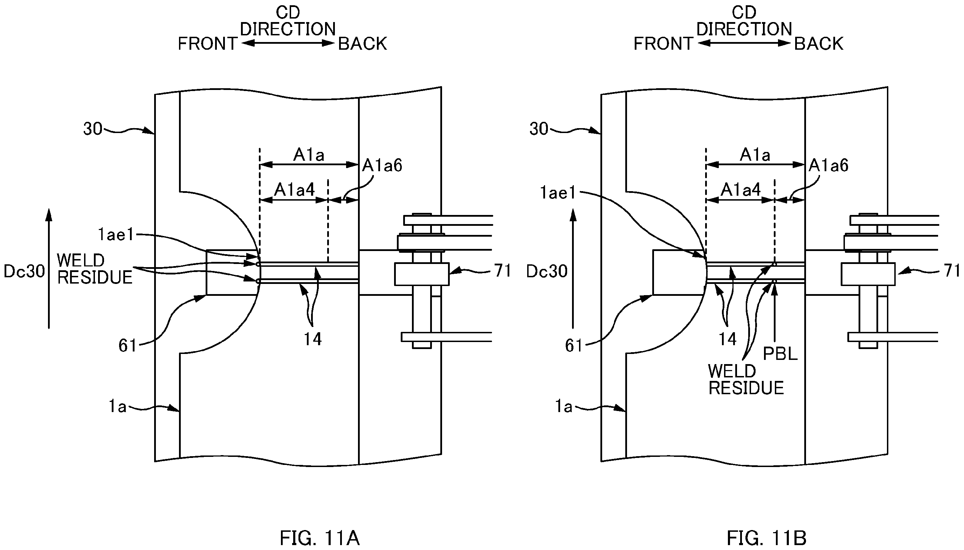

Such an operation can avoid a problem as illustrated in FIG. 11A that weld residue remains in a state of lying off the end edge part 1ae1 in the CD direction of the substrate 1a. The details are as follows.

First, since the anvil roller 71 moves in the CD direction during the ultrasonic welding process, melt formed by melting the substrate 1a in association with the welding process tends to be pushed out to the downstream side in the travelling direction of the anvil roller 71 moving in the CD direction. Thus, when the amount (J/m) of ultrasonic energy necessary for welding is applied throughout the crossing section A1a on the forward path as described above, such melt may protrude from the end edge part 1ae1 in the CD direction of the substrate 1a and harden, when the anvil roller 71 finishes crossing the crossing section A1a. Accordingly, such weld residue may remain at the end edge part 1ae1 in a burr manner, which may result in deterioration in appearance of the diaper 1.

In this regard, as described above, if the amount (J/m) of ultrasonic energy necessary for welding is applied only in the six-sheet section A1a6 on the forward path, and the amount (J/m) of ultrasonic energy necessary for welding is applied only in the four-sheet section A1a4 on the return path, melt remains at the boundary position PBL between the six-sheet section A1a6 and the four-sheet section A1a4 on the forward path, as illustrated in FIG 11B, while melt remains at the boundary position PBL similarly on the return path. Then, such melt hardens and results in formation of weld residue at the boundary position PBL. Thus, it becomes possible to effectively restrain such weld residue from protruding from the end edge part 1ae1 in the CD direction of the substrate 1a, resulting in favorable appearance of the diaper 1.

### <<< First Modified Example >>>

In the first embodiment mentioned above, the amplitude of the ultrasonic vibration is changed, so as to make the amount (J/m) of ultrasonic energy applied to the substrate 1a in the six-sheet section A1a6 on the forward path, different from the amount (J/m) of ultrasonic energy applied to the substrate 1a in the four-sheet section A1a4 on the same path. In this regard, in the first modified example, the amplitude is maintained constant without being changed between the six-sheet section A1a6 and the four-sheet section A1a4 on the forward path. And, instead thereof, a force with which the substrate 1a is sandwiched by the oscillating surface 61s of the horn 61 and the anvil roller 71, that is, the magnitude (N) of sandwiching force is changed between the six-sheet section A1a6 and the four-sheet section A1a4. As a result, the amount (J/m) of ultrasonic energy is made different between the six-sheet section A1a6 and the four-sheet section A1a4. This is a principal difference.

The rest is substantially the same as in the first embodiment. Therefore, such a difference will mainly be described below. The same components as those of the first embodiment will be denoted by the same reference numerals, and the descriptions thereof have been omitted.

First, in the first modified example, the ultrasonic-energy regulating unit controls the oscillator so that the amplitude of the ultrasonic vibration of the oscillating surface 61s of the horn 61 is maintained, for example, at a constant value of 30 µm. Further, the regulating unit controls the foregoing pressure-regulator valve for the air cylinder 75, which is provided in the support unit 73. According to such control, the substrate 1a is sandwiched with a greater sandwiching force (N) throughout the six-sheet section A1a6 on the forward path. On the other hand, the substrate 1a is sandwiched, with a sandwiching force (N) smaller than the sandwiching force in the six-sheet section A1a6, throughout the four-sheet section A1a4 on the forward path. As a result, the amount (J/m) of ultrasonic energy applied per unit length in the CD direction in the six-sheet section A1a6 is set greater than the amount (J/m) of ultrasonic energy applied per unit length in the CD direction in the four-sheet section A1a4.

For example, the magnitude (N) of sandwiching force in the four-sheet section A1a4 is set to any value within a range of 50% to 90% of the magnitude (N) of sandwiching force in the six-sheet section A1a6. It is desirable that the magnitude in the four-sheet section A1a4 is set to any value within a range of 70% to 80%. This makes it possible to reliably restrain defects such as a joint failure in the six-sheet section A1a6 and a melting loss in the four-sheet section A1a4.

In this example, the magnitude (N) of sandwiching force on the return path is made further smaller than the magnitude (N) of sandwiching force in the four-sheet section A1a4, so that the amount (J/m) of ultrasonic energy enough to avoid the substrate 1a from being welded is to be applied on the return path, however, it is not limited thereto. For example, this may be reversed. That is, on the forward path, the magnitude (N) of sandwiching force enough to avoid the substrate 1a from being welded may be set, while on the return path, the magnitude (N) of sandwiching force for welding of the substrate 1a may be set. Further, not only on the forward path but also the return path, the same magnitudes (N) of sandwiching forces as those in the four-sheet section A1a4 and the six-sheet section A1a6 on the forward path may be set in the sections A1a4 and A1a6 on the return path, respectively.

### <<< Second Modified Example >>>

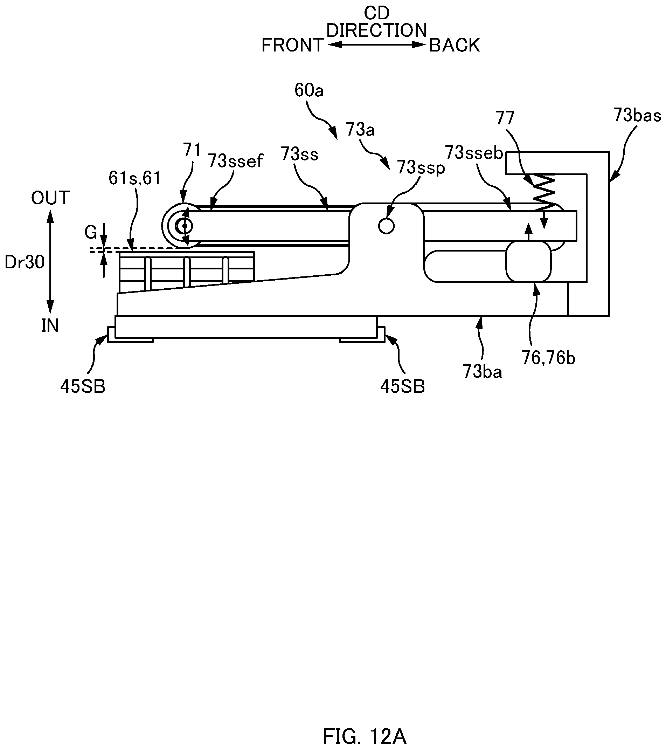

FIG. 12A is a schematic diagram of an ultrasonic processing unit 60a according to the second modified example as viewed in the rotation direction Dc30 of the rotating drum 30. In the second modified example, the size of the space G between the anvil roller 71 and the oscillating surface 61s of the horn 61 is changed, so as to make the amount (J/m) of ultrasonic energy different between the six-sheet section A1a6 and the four-sheet section A1a4 on the forward path.

That is, in the second modified example, the amplitude is also maintained at a constant value without being changed between the six-sheet section A1a6 and the four-sheet section A1a4 on the forward path, however, the size of the space G is changed between the six-sheet section A1a6 and the four-sheet section A1a4 on the forward path. Here, the sizes of the space G in the six-sheet section A1a6 and the four-sheet section A1a4 are obtained through actual experiments, etc. For more details, the ultrasonic welding processes are actually performed for the substrate 1a using the ultrasonic welding apparatus 20, with the sizes of the spaces G and G being varied in a plurality of levels. In association therewith, the welded parts 14 formed in the substrate 1a is visually checked, and thereby, the sizes of the spaces G and G are determined. Note that, in the visually checking, the presence or absence of a welding loss and a joint failure and the like in the substrate 1a is checked. In the case where the ultrasonic welding process is performed using the sizes of the spaces G for the six-sheet section A1a6 and the four-sheet section A1a4 determined as such, the amount (J/m) of ultrasonic energy applied per unit length in the CD direction in the six-sheet section A1a6 results in being greater than the amount (J/m) of ultrasonic energy applied per unit length in the four-sheet section A1a4.

Change in the space G is implemented, for example, with the following configuration. As shown in FIG. 12A, the second modified example also includes a support unit 73a having substantially the same configuration as that of the support units 73 in the first embodiment. That is, the anvil roller 71 is supported rotatably by the front-end part 73ssef of the seesaw-like member 73ss. And, as mentioned above, the seesaw-like member 73ss is also rotatably supported, with the support shaft 73ssp, onto the base section 73ba of the support unit 73a, the support shaft 73ssp being located at substantially the center in the CD direction. Accordingly, in the seesaw-like member 73ss, the front-end part 73ssef and the back-end part 73sseb can move in directions nearly opposite to each other. In other words, the seesaw-like member 73ss is capable of oscillating in a seesaw-like manner.

On the other hand, as driving sources for driving the seesaw-like member 73ss to oscillate, the support unit 73a of the second modified example includes: an air spring 76 that gives to the seesaw-like member 73ss a force in a direction in which the space G is reduced in size; and a leaf spring 77, serving as an elastic member, which gives to the seesaw-like member 73ss a force in a direction in which the space G is enlarged.

More specifically, the air spring 76 has a bag body 76b that is substantially sealed. The bag body 76b inflates when internally pressurized by an air supply, and deflates when internally depressurized by ejection of air. The bag body 76b is placed between the base section 73ba of the support unit 73a and the back-end part 73sseb of the seesaw-like member 73ss. And, the bag body 76b is in contact with the back-end part 73sseb from inside in the rotation-radius direction Dr30 of the rotating drum 30. The leaf spring 77 is supported by a suitable stay member 73bas, which is attached to the base section 73ba of the support unit 73a so as to be in contact with the back-end part 73sseb of the seesaw-like member 73ss from outside in the rotation-radius direction Dr30. Accordingly, the leaf spring 77 gives, to the back-end part 73sseb of the seesaw-like member 73ss, an elastic force directed inwards in the rotation-radius direction Dr30.

Accordingly, the bag body 76b is internally pressurized by such means as supplying air to the inside of the bag body 76b and increasing its supply pressure (MPa). Thereby, with inflation of the bag body 76b, the bag body 76b gives, to the abovementioned the back-end part 73sseb, a force directed outwards in the rotation-radius direction Dr30 of the rotating drum 30. When the magnitude of the force becomes greater than a force required to press and flatten the leaf spring 77 outwards in the rotation-radius direction Dr30, the seesaw-like member 73ss resists elastic force of the leaf spring 77 and simultaneously rotates so that the back-end part 73sseb moves outwards in the rotation-radius direction Dr30. And, the anvil roller 71 in the front-end part 73ssef moves inwards in the rotation-radius direction Dr30. Consequently, the space G between the oscillating surface 61s of the horn 61 and the anvil roller 71 is reduced.

On the other hand, the bag body 76b is internally depressurized by such means as decreasing supply pressure (MPa) at which air is supplied inside the bag body 76b. Thereby, with deflation of the bag body 76b, a force that the bag body 76b gives to the back-end part 73sseb is reduced. When such a force becomes smaller than a force required to press and flatten the leaf spring 77 outwards in the rotation-radius direction Dr30, the seesaw-like member 73ss rotates so that the back-end part 73sseb moves inwards in the rotation-radius direction Dr30. And, the anvil roller 71 in the front-end part 73ssef moves outwards in the rotation-radius direction Dr30, and the space G between the horn 61 and the anvil roller 71 is consequently enlarged.

A mechanism which supplies compressed air to the bag body 76b of the air spring 76 and which also ejects air from the same can be exemplified by a configuration in which a compressed-air source (e.g. a compressor; not shown) is connected to the bag body 76b through a supply channel (e.g. pipework; not shown), and in which a pressure-regulator valve (not shown) is arranged in the supply channel between the compressed-air source and the bag body 76b. In this configuration, the ultrasonic-energy regulating unit controls the pressure-regulator valve so as to change the size of the space G. However, the mechanism for supplying and ejecting compressed air is not limited thereto.

In the description above, the elastic member that gives a force in a direction in which the space G is enlarged is exemplified by the leaf spring 77. However, it is not limited thereto. For example, a disc spring or a coiled spring may also be employed. In addition, a bar-like member which undergoes elastic deflection deformation may be employed. For example, the one end section of the bar-like member is in contact with the back-end part 73sseb of the seesaw-like member 73ss from outside in the rotation-radius direction Dr30, and the bar-like member is supported, at two parts except for the one end section, by the base section 73ba of the support unit 73a so as not to rotate. When a force outwards in the rotation-radius direction Dr30 is applied to the back-end part 73sseb from the air spring 76, the bar-like member undergoes elastic deflection deformation, and thereby the size of the space G is changed.

### <<< Third Modified Example >>>

In the third modified example, the travel speed value (m/min) of the anvil roller 71 in the CD direction is changed, so that the amount (J/m) of ultrasonic energy is made different between the six-sheet section A1a6 and the four-sheet section A1a4 on the forward path. The detailed description is as follows.

First, the amplitude and the magnitude (N) of sandwiching force are maintained at the constant values, respectively, without being changed between the six-sheet section A1a6 and the four-sheet section A1a4 on the forward path. But, the travel speed value (m/min) of the anvil roller 71 in the CD direction is changed between the six-sheet section A1a6 and the four-sheet section A1a4 on the forward path. More specifically, the travel speed value (m/min) is set to be smaller in the six-sheet section A1a6 on the forward path, and the anvil roller 71 therefore moves at a low speed in the CD direction. On the other hand, the travel speed value (m/min) in the four-sheet section A1a4 on the forward path is set to be greater than the travel speed value (m/min) in the six-sheet section A1a6, and the anvil roller 71 therefore moves at a high speed in the CD direction. As a result, the amount (J/m) of ultrasonic energy applied per unit length in the CD direction in the six-sheet section A1a6 is made greater than the amount (J/m) of the ultrasonic energy applied in the four-sheet section A1a4.

Such a change in the travel speed value (m/min) of the anvil roller 71 in the CD direction is implemented by setting of the cam curve. That is, in this example, instead of the ultrasonic-energy regulating unit, which is used in the first embodiment and is configured with a computer or the like, the foregoing ribbed cam 51r and cam followers 53 and 53 serve as the ultrasonic-energy regulating unit. The detailed description is as follow.

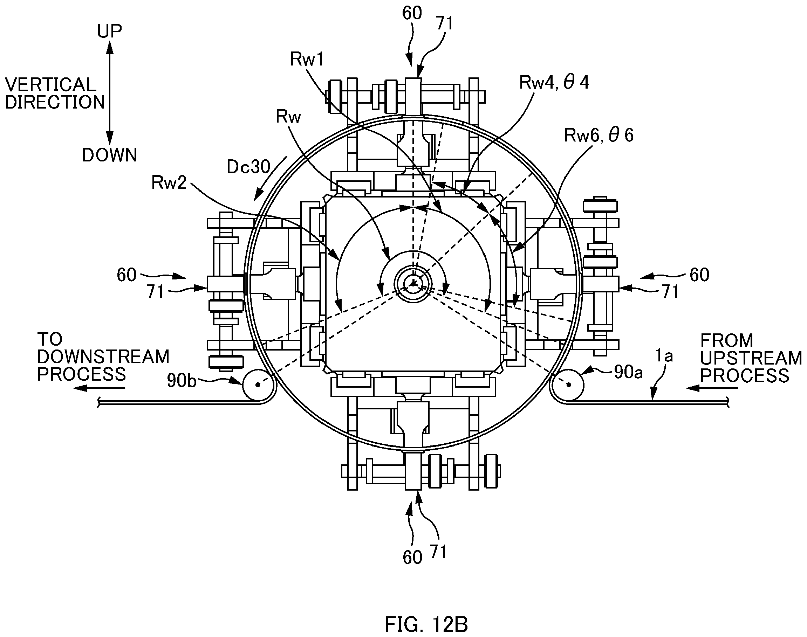

First, as mentioned above with reference to FIG. 8, in the cam curve of the cam 51r, the movement in the forward path (forward movement) is set corresponding to the first angular range Rw1 in the rotation direction Dc30 of the rotating drum 30. Such a forward movement includes a movement in the six-sheet section A1a6 and a movement in the four-sheet section A1a4. Thus, as shown in FIG. 12B, when an angular range Rw6 of the six-sheet section A1a6 and an angular range Rw4 of the four-sheet section A1a4 are assigned to the first angular range Rw1, a value (=θ6/θ4) obtained by dividing the magnitude θ6 of the angular range Rw6 in the six-sheet section A1a6 by the magnitude θ4 of the angular range Rw4 in the four-sheet section A1a4 is set to be greater than a value (L6/L4) obtained by dividing a length L6 in the CD direction in the six-sheet section A1a6 in FIG. 10A by a length L4 in the CD direction in the four-sheet section A1a4. With such a setting being made, the travel speed value (m/min) in the six-sheet section A1a6 can be made smaller than the travel speed value (m/min) in the four-sheet section A1a4.

In the first embodiment and the first to the third modified examples, the rail-like horn 61 is arranged so as not to move relative to the rotating drum 30, and the roller-like anvil 71, which serves as the anvil roller 71, is arranged outside, in the rotation-radius direction Dr30, with respect to the horn 61. However, it is not limited thereto. For example, the following configuration may be employed. That is, a rail-like anvil extending in the CD direction is fixed so as not to move relative to the rotating drum 30, and simultaneously a roller-like horn (hereinafter referred to as a horn roller) may be arranged outside, in the rotation-radius direction Dr30, with respect to the anvil. The configuration of the horn roller is the same as disclosed in Japanese Unexamined Patent Application Publication (Translation of PCT Application) No. 10-513128, and the configuration is known. Accordingly, the detailed description will be omitted.

### === Second Embodiment ===

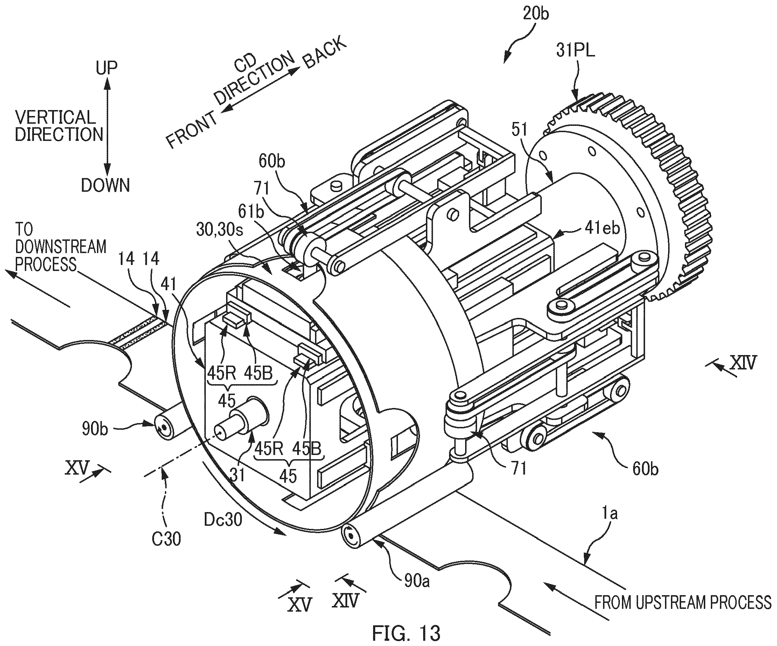

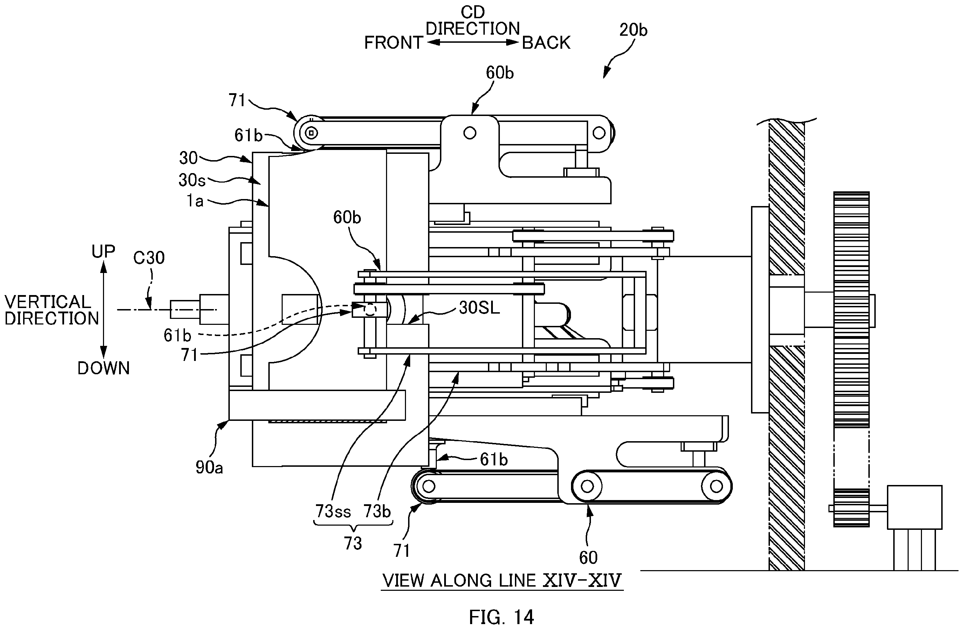

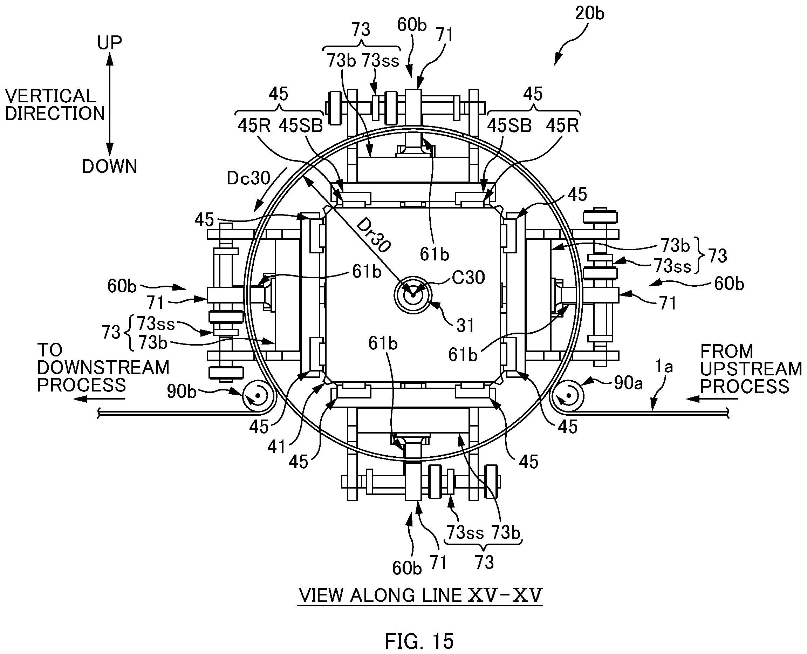

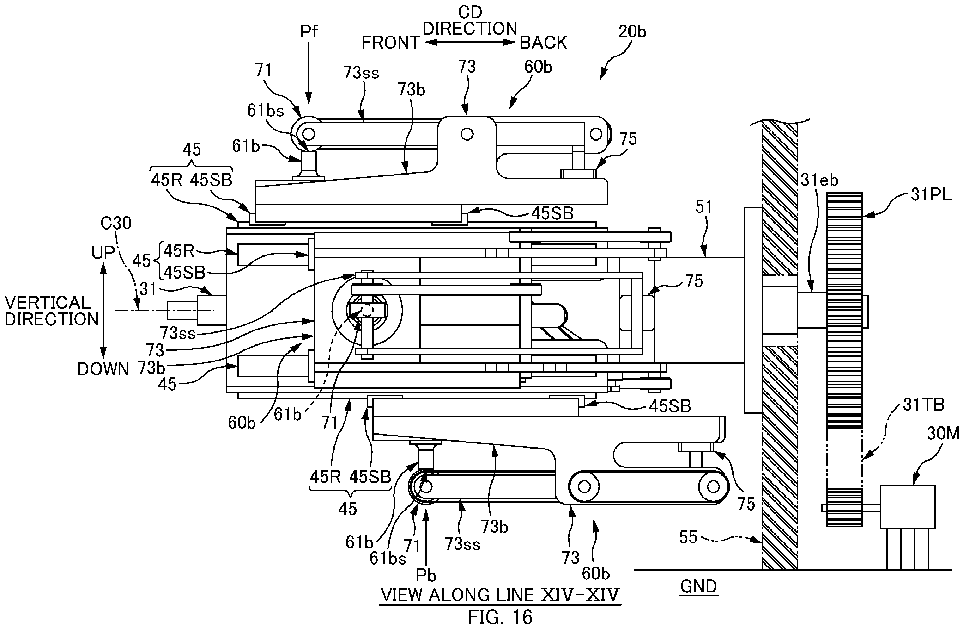

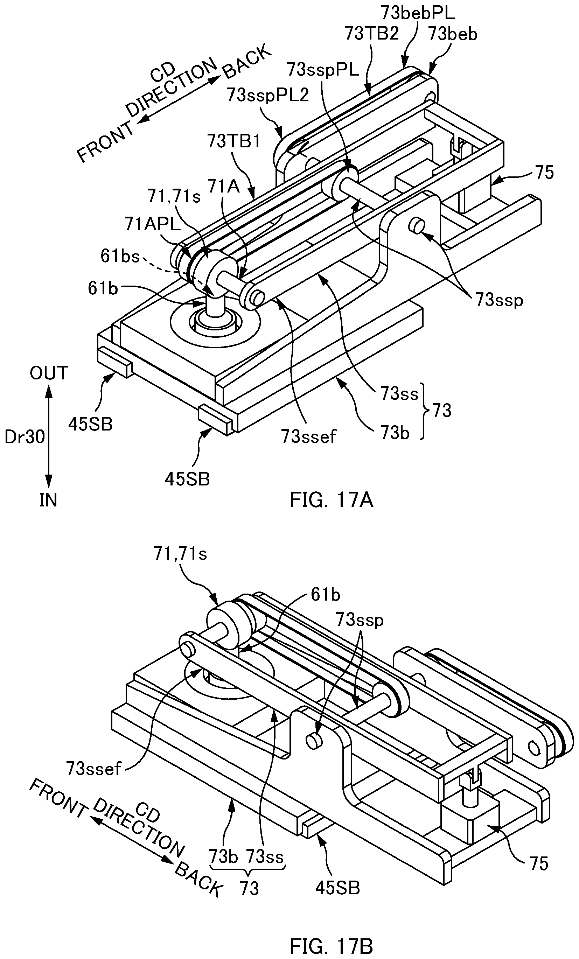

FIGS. 13 to 17B are diagrams illustrating the ultrasonic welding apparatus 20b according to the second embodiment. FIG. 13 is a schematic perspective view of the ultrasonic welding apparatus 20b as viewed obliquely from front above. FIG. 14 is a schematic side view along arrows XIV-XIV in FIG. 13, and FIG. 15 is a schematic front view along arrows XV-XV in FIG. 13. FIG. 16 is a schematic side view obtained by removing the substrate 1a and the rotating drum 30 from FIG. 14. FIG. 17A is a schematic perspective view of the ultrasonic processing unit 60b as viewed obliquely from front and outside in the rotation-radius direction Dr30, and FIG. 17B is a schematic perspective view of the unit 60b as viewed obliquely from back and outside in the rotation-radius direction Dr30.

In the first embodiment mentioned above, the horn 61 of the ultrasonic processing unit 60 is fixed to the column 41 so as not to move relative to the rotating drum 30, as shown in FIG. 5. Thus, the horn 61 does not reciprocate in the CD direction, as shown in FIG. 6. In this regard, the second embodiment is different from the first embodiment mainly in that a horn 61b reciprocates in the CD direction along with the reciprocation in the CD direction of the anvil roller 71, as shown in FIG. 16. The rest of the configuration is substantially the same as that of the first embodiment. Therefore, such a difference will mainly be described below. The same components as those of the first embodiment will be denoted by the same reference numerals, and the description thereof has been omitted.

As shown in FIG. 16, in the second embodiment, not only the anvil roller 71 but also the horn 61b is supported by the support unit 73. Thereby, the horn 61b is also capable of reciprocating in the CD direction. The anvil roller 71 and the horn 61b are configured to cooperate with each other so that the substrate 1a can be sandwiched therebetween. For example, in this example, the horn 61b is fixed to the base section 73b of the support unit 73. On the other hand, the anvil roller 71 is supported by the seesaw-like member 73ss. Accordingly, both of the anvil roller 71 and the horn 61b reciprocate in the CD direction, while ultrasonic energy is being applied to the substrate 1a from the oscillating surface 61bs of the ultrasonically vibrating horn 61b, in a state where the anvil roller 71 and the oscillating surface 61bs of the horn 61b cooperate with each other so that the substrate 1a is sandwiched therebetween. Consequently, a welded part 14 along the CD direction is formed in the substrate 1a.

In the ultrasonic welding process to the substrate 1a, weld residue may adhere to and is accumulated on the horn 61b in particular; this is because the horn 61b does not rotate. But, in this example, not only the anvil roller 71 but also the horn 61b reciprocates. Accordingly, during its reciprocation, the horn 61b can wipe successively the weld residue attached thereto, by being in contact with the substrate 1a and by sliding on the substrate 1a. This makes it possible to effectively prevent accumulation of weld residue on the horn 61b.

A movement of sandwiching the substrate 1a between the anvil roller 71 and the oscillating surface 61bs of the horn 61b is implemented by the movement of the anvil roller 71 in the rotation-radius direction Dr30 of the rotating drum 30 through oscillation of the seesaw-like member 73ss, the oscillation caused by mainly the air cylinder 75. Accordingly, such a movement can be performed without moving the horn 61b in the rotation-radius direction Dr30 of the rotating drum 30. This makes it possible to stabilize ultrasonic vibration of the horn 61b, which is configured with precision devices.

Also in the second embodiment, as shown in FIG. 14, the main body of the rotating drum 30 is a cylinder 30, which forms the outer circumferential face 30s. But, on the cylinder 30, slit-shaped notches 30SL are formed extending from back to front in the CD direction, and the notches 30SL correspond to reciprocation paths in the CD direction of each pair of the anvil roller 71 and the horn 61b. Accordingly, a space is formed at the position of each notch 30SL. That is, the inside of the cylinder 30 communicates with the outside of the cylinder. Accordingly, the anvil roller 71 and the horn 61b can reciprocate quickly in the CD direction without any interference with the cylinder 30, while the substrate 1a is being sandwiched, at the position of the notch 30SL, between the anvil roller 71 and the horn 61b from both sides in the rotation-radius direction Dr30 of the rotating drum 30.

In the second embodiment, since the horn 61b reciprocates in the CD direction together with the anvil roller 71, it is not necessary for the horn 61b to have a rail-like shape extending in the CD direction as described in the first embodiment. That is, the shape of the horn 61b may be selected freely. Accordingly, in the second embodiment, the shape of the horn 61b is a substantially cylindrical body 61b whose axial direction is oriented in the rotation-radius direction Dr30 of the rotating drum 30, as shown in FIG. 17A. A circular side surface 61bs of the substantially cylindrical body 61b serves as the oscillating surface 61bs that vibrates ultrasonically, and the side surface 61bs faces outwards in the rotation-radius direction Dr30 of the rotating drum 30. Consequently, the horn 61b is fixed to the base section 73b of the support unit 73 with its side surface 61bs facing the circumferential face 71s of the anvil roller 71.

The basic configuration of the ultrasonic welding apparatus 20b according to the second embodiment is substantially the same as that of the apparatus disclosed in Japanese Unexamined Patent Application Publication No. 2013-193450. And, the detailed description thereof has been omitted.

Based on the foregoing description, implementation can be considered possible for persons skilled in the art by applying the configurations of the first to third modified examples of the first embodiment to the ultrasonic welding apparatus 20b of the second embodiment. Thus, the description thereof has been omitted.

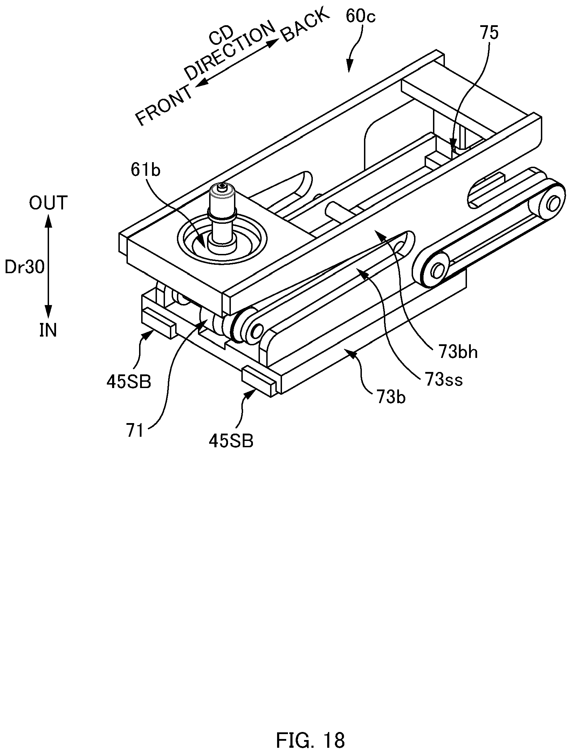

In addition, in the second embodiment, as shown in FIG. 17A, the horn 61b having a substantially cylindrical body is arranged inside, in the rotation-radius direction Dr30 of the rotating drum 30, with respect to the anvil roller 71. However, it is not limited thereto. For example, the positional relation in the rotation-radius direction Dr30 may be inverted. That is, the anvil roller 71 may be arranged inside, in the rotation-radius direction Dr30, with respect to the horn 61b substantially of a cylindrical body. In this case, the front-end part 73ssef of the seesaw-like member 73ss supports the horn 61b, and the base section 73b supports the anvil roller 71. However, in some cases, an ultrasonic processing unit may be the ultrasonic processing unit 60c shown in the schematic perspective view of FIG. 18. That is, in this example, the anvil roller 71 is supported by the seesaw-like member 73ss. The foregoing base section 73b includes an extending part 73bh, which extends outwardly in the rotation-radius direction Dr30 beyond the position of the seesaw-like member 73ss. And, the extending part 73bh supports the horn 61b. Such a configuration may be employed.

### === Other Embodiments ===

While the embodiments of the invention are described above, the embodiments are for the purpose of elucidating the understanding of the invention and are not to be interpreted as limiting the invention. The invention can of course be altered and improved without departing from the gist thereof, and equivalents are intended to be embraced therein. The invention can be altered as described below, for example.

In the first embodiment, for the purpose of explanation, the description is made based on an assumption that the circumferential speed value (m/min) of the rotating drum 30 is constant. But, as mentioned above, the circumferential speed value of the rotating drum 30 may change in actual use. In such a case, with the amplitude of the ultrasonic vibration being increased/decreased in conjunction with increase/decrease in the circumferential speed value (m/min) of the rotating drum 30, the magnitude relationship between the amount (J/m) of ultrasonic energy applied per unit length in the CD direction in the six-sheet section A1a6 and the amount (J/m) of ultrasonic energy applied per unit length in the CD direction in the four-sheet section A1a4 can be maintained, without being affected by change in the circumferential speed value (m/min).

In the first embodiment, as shown in FIG. 9, the anvil roller 71 includes two ribs 71r and 71r on the circumferential face 71s, and a plurality of protrusions 71p, 71p... are provided in a pattern on the top surface 71rs of each rib 71r. However, it is not limited thereto. For example, the following configuration may be employed: two ribs along the CD direction are provided on the oscillating surface 61s of the rail-like horn 61 illustrated in FIG. 6, and a plurality of protrusions are provided in a pattern on the top surface of each rib. In some cases, the abovementioned ribs 71r and protrusions 71p, 71p... may be provided on both of the anvil roller 71 and the horn 61. Further, the rib 71r is not essential, that is, a plurality of protrusions 71p, 71p... may be provided in a pattern directly on the circumferential face 71s of the anvil roller 71 or the oscillating surface 61s of the horn 61.

In the foregoing embodiment, as an example of the absorbent article, a disposable diaper 1 is described which is attached to a wearer and absorbs excretion liquid. However, it is not limited thereto. That is, anything which absorbs excretion liquid such as urine or menstrual blood may be employed as an absorbent article according to the present invention. For example, a sanitary napkin and a pet pad absorbing excretion liquid of pets are also included in the concept of an absorbent article according to the present invention.

In the foregoing embodiment, such an example is described in which the substrate 1a includes the four-sheet stacked part 1a4 and the six-sheet stacked part 1a6, as the parts 1a4 and 1a6 having the numbers of stacked sheets different from each other. However, the combination of the numbers of stacked sheets is not limited to four sheets and six sheets. For example, other combinations may be employed, such as two sheets and four sheets, six sheets and eight sheets, and the like. Further, such parts having the numbers of stacked sheets different from each other may not necessarily be two parts as described above, but may be three parts or more.

In the foregoing embodiment, the substrate 1a includes the four-sheet stacked part 1a4 and the six-sheet stacked part 1a6 as the parts 1a4 and 1a6 having the numbers of stacked sheets different from each other. In association therewith, the four-sheet section A1a4 and the six-sheet section A1a6 are set in the crossing section A1a. Further, the amount (J/m) of ultrasonic energy to be applied is made different from each other between the four-sheet section A1a4 and the six-sheet section A1a6. However, it is not limited thereto.

For example, as simply described in the beginning of the present specification, in the case where a plurality of parts 1P1BH, 1P1C, and 1P1LH, having weld strength different from each other are formed in the substrate 1a, the substrate 1a may not necessarily include a plurality of parts having the numbers of stacked sheets different from each other. That is, also in this case, a plurality of sections A1aB, A1aC, and A1aL are set in the crossing section A1a corresponding to the abovementioned plurality of parts 1P1BH, 1P1C, and 1P1LH, as well as the amount (J/m) of ultrasonic energy to be applied is made different from each other among the sections A1aB, A1aC, and A1aL. Specific description will be described as follows.

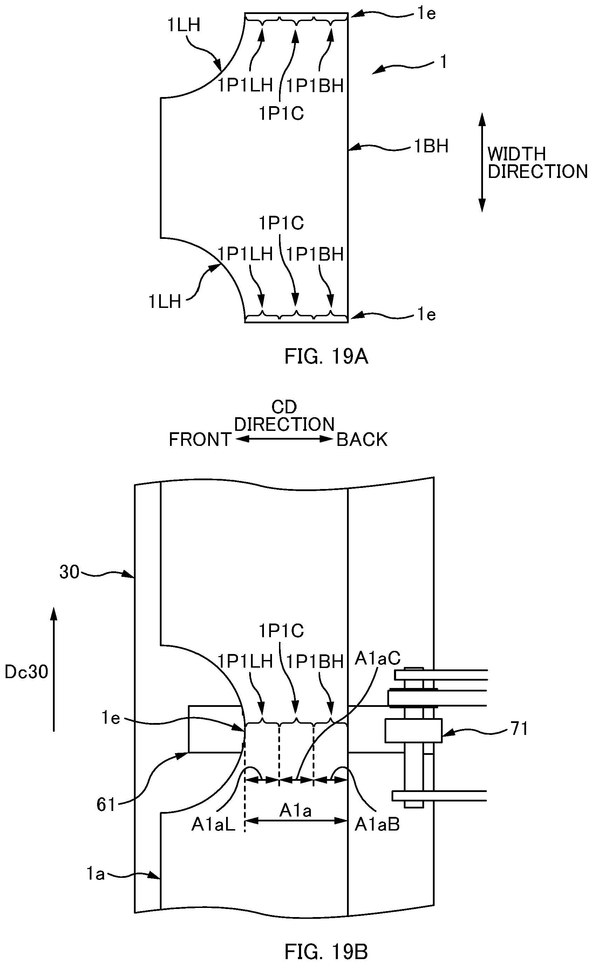

First, as illustrated in the schematic plan view of the diaper 1 in FIG. 19A, each of the side-end parts 1e in the width direction, in which the abovementioned welded parts 14 are formed in the diaper 1, is divided into three, which are the waist-opening part 1P1BH; the leg opening part 1P1LH; and the central part 1P1C arranged between these parts 1P1BH and 1P1LH. In this case, the waist-opening part 1P1BH and the leg opening part 1P1LH are subject to relatively greater external force, since the leg openings 1LH and 1LH and waist opting 1BH are expanded or the like when the diaper is worn or the like. Thus, greater weld strength is necessary. Whereas, since the central part 1P1C is subject to relatively smaller external force, lower weld strength is enough in the central part 1P1C.

Further, as illustrated in FIG. 19B, in the substrate 1a of the diaper 1 wound around the rotating drum 30, the abovementioned waist-opening part 1P1BH (corresponding to the first part) and leg opening part 1P1LH (corresponding to the first part) are located on the both side parts in the CD direction, respectively, and the central part 1P1C (corresponding to the second part) is located in the center in the CD direction. Thus, in this case, first, the crossing section A1a is divided into three sections A1aB, A1aC, and A1aL in the CD direction, corresponding to these parts 1P1BH, 1P1LH, and 1P1C. Then, when the anvil roller 71 passes through the sections A1aB, A1aC, and A1aL, the ultrasonic energy is applied with the amount (J/m) of ultrasonic energy corresponding to each of the sections A1aB, A1aC, and A1aL. That is, in the section A1aB (corresponding to the first section) and the section A1aL (corresponding to the first section), the amount (J/m) of ultrasonic energy greater than that in the section A1aC (corresponding to the second section) is to be applied. From the above, it is clear that a target to be processed by the ultrasonic welding apparatuses 20, and 20b of the present invention is not limited to the substrate 1a including a plurality of parts 1a4 and 1a6 having the number of stacked sheets different from each other.

In the foregoing embodiment, a rotating member is exemplified by the rotating drum 30. However, it is not limited thereto. That is, it is possible to use, without any problem, a member which rotatable along the outer circumferential face 30s while holding the substrate 1a of the absorbent article 1 on the outer circumferential face 30s.

In the foregoing embodiment, as shown in FIG. 3, the substrate 1a, which is an example of the sheet-like member 1a, is a continuous body in the transport direction. However, it is not limited thereto. For example, the substrate 1a may be a single-cut member having a size corresponding to a single diaper. However, in this case, it is preferable that a mechanism for actively holding the substrate 1a is provided on the outer circumferential face 30s of the rotating drum 30. For example, it is preferable that a plurality of suction holes are formed on the outer circumferential face 30s of the rotating drum 30 to attach the substrate 1a onto outer circumferential face 30s by sucking from the suction holes.

In the first embodiment, as shown in FIG. 6, a driving mechanism that reciprocates, in the CD direction, the support unit 73 associated with the anvil roller 71 is exemplified by the cam mechanism. The cam mechanism is exemplified by the followings: the ribbed cam 51r provided on the outer circumferential face 51s of the cylindrical member 51; and a pair of cam followers 53 and 53 which is provided in the base section 73b of the support unit 73 and which engages with the cam 51r by sandwiching it therebetween in the front-back direction. However, it is not limited thereto. For example, the following configuration is also acceptable: an endless grooved cam is provided based on the foregoing cam curve on the outer circumferential face 51s of the cylindrical member 51; a cam follower is provided on the base section 73b; and the cam follower fits into the grooved cam and engages with it.

As shown in FIG. 10A, the forward path and the return path of the reciprocation in the CD direction includes sections Aef and Aeb except for the crossing section A1a. That is, in the forward path and the return path, the sections Aef and Aeb are located closer to the ends in the CD direction than the crossing section A1a is. In other words, in the forward path and the return path, the substrate 1a does not exist in the sections Aef and Aeb. The first embodiment does not describe in detail the magnitude (N) of sandwiching force by the horn 61 and the anvil roller 71 in the sections Aef and Aeb, however, the magnitude (N) of pressing force to the horn 61 (the foregoing sandwiching force) may be set to zero, by separating the anvil roller 71 from the horn 61 in the sections Aef and Aeb. A method for separating them can be exemplified as follows: when the anvil roller 71 has reached a position close to an end in the CD direction with respect to the crossing section A1a, the anvil roller 71 is moved by a cam mechanism outwards in the rotation-radius direction Dr30 of the rotating drum 30, the cam mechanism being additionally provided in the outer circumferential face 30s of the rotating drum 30. This separation operation makes it possible to effectively prevent the followings: damage or wear of components of the ultrasonic processing apparatus 20 caused by metallic contact between the anvil roller 71 and the horn 61 that vibrates ultrasonically; contamination of the substrate 1a caused by generation of wear metal powder; and the like.

In the foregoing embodiment, the CD direction, which intersects the transport direction of the substrate 1a, is exemplified by a direction orthogonal to the transport direction. However, it is not limited thereto. That is, the CD direction may be slightly inclined to the direction orthogonal to the transport direction.

In the foregoing embodiment, the dimensions in the transport direction (rotation direction Dc30) according to the welded part 14, that is, the dimension in the transport direction of high-compressed portions and the dimension in the transport direction of the low-compressed portions are set at a predetermined value throughout the CD direction. However, it is not limited thereto, and the dimensions may vary.

In the foregoing embodiment, a section, in which the anvil roller 71 crosses the substrate 1a along the CD direction, on the outer circumferential face 30s of the rotating drum 30 serving as a rotating member, is defined as the "crossing section A1a" (see FIG. 10A). Here, this crossing section A1a is supplementary described as follows : the crossing section A1a means "the crossing section A1a corresponding to a section from the position at which the anvil roller 71 starts crossing the substrate 1a in the CD direction to the position at which the roller finishes crossing, in a state where the substrate 1a is wound around in a designed position of the outer circumferential face 30s of the rotating drum 30 without meandering." That is, the crossing section A1a is "the section A1a sandwiched between a back end edge part 1aeb and a front end edge part 1aef in the CD direction of the substrate 1a in a state in which the substrate 1a is wound in the above designed position."

### [Reference Signs List]

1 disposable diaper (absorbent article), 1BH waist opening, 1LH leg opening,
1P1BH waist-opening part (first part),
1P1LH leg opening part (first part),
1P1C central part (second part),
1a substrate (sheet-like member),
1a4 four-sheet stacked part (second part), 1a6 six-sheet stacked part (first part),
1ae end edge part, 1ae1 end edge part, 1aef front end edge, 1aeb back end edge,
1ap part, 1c part,
1e side-end part (to-be-welded part),
2a continuous sheet,
2a1 inner-layer sheet, 2a1e end,
2a2 outer-layer sheet, 2a2e part,
2ae end part (three-sheet stacked part), 2an two-sheet stacked part,
4 absorbent main body,
6 leg-circumference elastic member, 7 waist-circumference elastic member,
10 front piece, 11 back piece, 13 crotch part,
14 welded part,
20 ultrasonic processing apparatus, 20b ultrasonic welding apparatus,
30 rotating drum (rotating member, cylinder), 30s outer circumferential face, 30SL notch,
30M servomotor (driving source), 30MPL pulley,
30TB timing belt,
31 shaft member, 31Brg bearing member,
31PL pulley, 31eb one end section,
41 column, 41eb one end section, 41w wall section,
45 linear guide, 45R rail, 45SB sliding block,
51 cylindrical member, 51s outer circumferential face,
51r ribbed cam, 53 cam follower,
55 support member on ground side,
60 ultrasonic processing unit, 60a ultrasonic processing unit,
60b ultrasonic processing unit, 60c ultrasonic processing unit,
61 horn (rail-like member, ultrasonic processing member), 61s oscillating surface,
61b horn (ultrasonic processing member), 61bs side surface (oscillating surface),
71 anvil roller (anvil, ultrasonic processing member),
71A shaft section, 71APL pulley,
71s circumferential face, 71r rib, 71rs top surface,
71p protrusion, 71ps top surface,
73 support unit, 73a support unit,
73TB1 timing belt, 73TB2 timing belt,
73b base section, 73ba base section,
73bas stay member,
73beb back-end part, 73bebPL pulley,
73bh extending part,
73ss seesaw-like member, 73ssef front-end part, 73sseb back-end part,
73ssp support shaft,
73sspPL another pulley, 73sspPL2 another pulley,
75 air cylinder, 75c cylinder section, 75pr piston rod,
76 air spring, 76b bag body,
90a guide roll, 90b guide roll,
A1a crossing section,
A1a4 four-sheet section (second section), A1a6 six-sheet section (first section),
A1aB section (first section), A1aC section (second section),
A1aL section (first section),
Aef section of path except for crossing section, Aeb section of path except for crossing section,
Pf forward limit, Pb backward limit,
PBL boundary position,
Rw winding angular range, Rw1 first angular range, Rw2 second angular range,
Rw3 angular range except for first and second angular ranges, Pws winding start position, Pwe winding end position,
G space GND ground,
C30 central axis, C71 rotational axis,

## Claims

1. An ultrasonic welding apparatus (20) that performs an ultrasonic welding process to a sheet-like member (1a), the sheet-like member being associated with an absorbent article, the sheet-like member being transported while being wound onto an outer circumferential face (30s) of a rotating member (30), the rotating member rotating about its central axis (C30), the apparatus comprising:
the rotating member (30); and
an ultrasonic processing unit (60)
that rotates about the central axis (C30) together with the rotating member, and
that includes, as ultrasonic processing members,
a horn (61) that vibrates ultrasonically and
an anvil (71) between which and the horn
the sheet-like member is sandwiched;
at least either one ultrasonic processing member of the horn and the anvil being guided so as to reciprocate in a CD direction, the CD direction intersecting a transport direction of the sheet-like member,
the at least either one ultrasonic processing member of the horn and the anvil reciprocating in the CD direction with respect to a part of the sheet-like member, the part being wound around the rotating member,
the outer circumferential face (30s) of the rotating member (30) including a first section (A1a6) and a second section (A1a4) at positions different from each other in the CD direction,
**characterized in that** the apparatus further comprises an ultrasonic-energy regulating unit,
the ultrasonic-energy regulating unit making
an amount (J/m) of ultrasonic energy applied per unit length in the CD direction when the ultrasonic processing member passes through the first section on either one of a forward path and a return path of the reciprocation
different from
an amount (J/m) of ultrasonic energy applied per unit length in the CD direction when the ultrasonic processing member passes through the second section on either one of the forward path and the return path.

2. An ultrasonic welding apparatus of a sheet-like member associated with an absorbent article according to claim 1, wherein
when the ultrasonic processing member passes through the first section (A1a6) and the second section (A1a4) on one of the forward path and the return path of the reciprocation, a first part (1a6) corresponding to the first section in the sheet-like member is welded by applying an amount (J/m) of the ultrasonic energy corresponding to the first part, and a second part (1a4) corresponding to the second section in the sheet-like member is welded by applying an amount (J/m) of the ultrasonic energy corresponding to the second part.

3. An ultrasonic welding apparatus of a sheet-like member associated with an absorbent article according to claim 1, wherein
when the ultrasonic processing member passes through the first section (A1a6) on one of the forward path and the return path of the reciprocation, a first part (1a6) corresponding to the first section in the sheet-like member is welded by applying an amount (J/m) of the ultrasonic energy corresponding to the first part,
when the ultrasonic processing member passes through the second section (A1a4) on an other of the forward path and the return path of the reciprocation, a second part (1a4) corresponding to the second section in the sheet-like member is welded by applying an amount (J/m) of the ultrasonic energy corresponding to the second part, and
on the forward path, the ultrasonic processing member passes through the first section and thereafter the second section, and the second part (1a4) is not welded in the second section on the forward path,
on the return path, the ultrasonic processing member passes through the second section and thereafter the first section, and the first part (1a6) is not welded in the first section on the return path.

4. An ultrasonic welding apparatus of a sheet-like member associated with an absorbent article according to any one of claims 1 to 3, wherein
the sheet-like member (1a) includes a plurality of sheets stacked in a thickness direction,
the sheet-like member includes a plurality of parts having the number of stacked sheets different from each other, the plurality of parts being arranged in the CD direction, and
assuming that a part having the greater number of stacked sheets in the plurality of parts is a first part (1a6), and a part having the number of stacked sheets smaller than the number thereof in the first part is a second part (1a4),
the first section (A1a6) corresponds to the first part, and the second section (A1a4) corresponds to the section part, and
an amount (J/m) of the ultrasonic energy applied per unit length in the first section is greater than an amount (J/m) of the ultrasonic energy applied per unit length in the second section.

5. An ultrasonic welding apparatus of a sheet-like member associated with an absorbent article according to any one of claims 1 to 3, wherein
the sheet-like member (1a) includes a plurality of parts between or among which weld strength is to be made different from each other, the plurality of parts being arranged in the CD direction, and
assuming that a part that is to have greater weld strength in the plurality of parts is a first part (1a6), and a part that is to have weld strength smaller than the weld strength in the first part is a second part (1a4),
the first section (A1a6) corresponds to the first part, and the second section (A1a4) corresponds to the second part, and
an amount (J/m) of the ultrasonic energy applied per unit length in the first section is greater than an amount (J/m) of the ultrasonic energy applied per unit length in the second section.

6. An ultrasonic welding apparatus of a sheet-like member associated with an absorbent article according to any one of claims 1 to 5, wherein
the ultrasonic-energy regulating unit changes an amount (J/m) of the ultrasonic energy applied per unit length, by changing an amplitude of ultrasonic vibration of the horn.

7. An ultrasonic welding apparatus of a sheet-like member associated with an absorbent article according to any one of claims 1 to 5, wherein
the ultrasonic-energy regulating unit changes an amount (J/m) of the ultrasonic energy applied per unit length, by changing a magnitude of sandwiching force with which the sheet-like member is sandwiched between the horn (61) and the anvil (71).

8. An ultrasonic welding apparatus of a sheet-like member associated with an absorbent article according to any one of claims 1 to 5, wherein
the ultrasonic-energy regulating unit changes an amount (J/m) of the ultrasonic energy applied per unit length, by changing a size of a space (G) between the horn (61) and the anvil (71).

9. An ultrasonic welding apparatus of a sheet-like member associated with an absorbent article according to any one of claims 1 to 5, wherein
the ultrasonic-energy regulating unit changes an amount (J/m) of the ultrasonic energy applied per unit length, by changing a travel speed value at which the ultrasonic processing member moves in the CD direction.

10. An ultrasonic welding apparatus of a sheet-like member associated with an absorbent article according to any one of claims 1 to 9, wherein
the ultrasonic processing member that is either one of the horn (61) and the anvil (71) includes a roller member, the roller member being provided rotatably and arranged outside with respect to the outer circumferential face of the rotating member, and
the roller member moves in the CD direction while rolling on a rail-like member, the rail-like member being provided extending in the CD direction not to move relative to the outer circumferential face of the rotating member, the rail-like member serving as the other ultrasonic processing member.

11. An ultrasonic welding apparatus of a sheet-like member associated with an absorbent article according to any one of claims 1 to 9, wherein
both of the horn (61) and the anvil (71) reciprocate in the CD direction while the sheet-like member (1a) is being sandwiched between the horn and the anvil.

12. An ultrasonic welding method in which a sheet-like (1a) member associated with an absorbent article is subject to an ultrasonic welding process, the sheet-like member being transported while being wound onto an outer circumferential face (30s) of a rotating member (30), the rotating member rotating about its central axis (C30), the method comprising:
rotating an ultrasonic processing unit (60) about the central axis (C30) together with the rotating member (30), the ultrasonic processing unit including, as ultrasonic processing members, a horn (61) and an anvil (71) facing the horn;
causing the horn (61) to vibrate ultrasonically; and
causing at least either one ultrasonic processing member of the horn and the anvil to reciprocate in a CD direction with respect to a part of the sheet-like member, the CD direction intersecting a transport direction of the sheet-like member, while the sheet-like member is sandwiched between the horn and the anvil, the part being wound around the rotating member,
the outer circumferential face (30s) of the rotating member (30) including a first section (A1a6) and a second section (A1a4) at positions different from each other in the CD direction,
**characterized in** an amount (J/m) of ultrasonic energy applied per unit length in the CD direction when the ultrasonic processing member passes through the first section (A1a6) on either one of a forward path and a return path of the reciprocation being made different from an amount (J/m) of ultrasonic energy applied per unit length in the CD direction when the ultrasonic processing member passes through the second section (A1a4) on either one of the forward path and the return path.

## Patentansprüche

1. Ultraschallschweißvorrichtung (20), die ein Ultraschallschweißverfahren an einem lagenartigen Element (1a) ausführt, wobei das lagenartige Element mit einem absorbierenden Artikel in Verbindung steht, das lagenartige Element transportiert wird, während es auf eine äußere Umfangsfläche (30s) eines rotierenden Elements (30) gewickelt wird, das rotierende Element um seine Mittelachse (C30) rotiert, wobei die Vorrichtung Folgendes umfasst:
das rotierende Element (30); und
eine Ultraschallverarbeitungseinheit (60),
die um die Mittelachse (C30) zusammen mit dem rotierenden Element rotiert und
die als Ultraschallverarbeitungseinheiten Folgendes einschließt:
ein Horn (61), das mit Ultraschall vibriert und
einen Amboss (71), zwischen dem und dem Horn das lagenartige Element eingeklemmt wird;
wobei mindestens ein Ultraschallverarbeitungselement von dem Horn und dem Amboss in einer Hinundherbewegung in einer CD-Richtung geführt wird, wobei die CD-Richtung eine Transportrichtung des lagenartigen Elements überkreuzt,
sich das mindestens eine Ultraschallverarbeitungselement von dem Horn und dem Amboss in der CD-Richtung in Bezug auf ein Teil des lagenartigen Elements hin- und herbewegt, das Teil um das rotierende Element gewickelt wird,
die äußere Umfangsfläche (30s) des rotierenden Elements (30) einen ersten Abschnitt (A1a6) und einen zweiten Abschnitt (A1a4) an Positionen, die sich in der CD-Richtung voneinander unterscheiden, einschließt,
**dadurch gekennzeichnet, dass** die Vorrichtung weiter eine Ultraschallenergie regulierende Einheit umfasst,
die Ultraschallenergie regulierende Einheit Folgendes erzeugt:
eine Menge (J/m) an Ultraschallenergie, die pro Einheitslänge in der CD-Richtung angewendet wird, wenn das Ultraschallverarbeitungselement durch den ersten Abschnitt auf einem von einem Vorwärtsweg und einem Rückwärtswegs der Hinundherbewegung läuft,
die sich unterscheidet von
einer Menge (J/m) an Ultraschallenergie, die pro Einheitslänge in der CD-Richtung angewendet wird, wenn das Ultraschallverarbeitungselement durch den zweiten Abschnitt auf einem von dem Vorwärtsweg und dem Rückwärtsweg läuft.

2. Ultraschallschweißvorrichtung eines lagenartigen Elements, das mit einem absorbierenden Artikel in Verbindung steht, nach Anspruch 1, wobei,
wenn das Ultraschallverarbeitungselement durch den ersten Abschnitt (A1a6) und den zweiten Abschnitt (A1a4) auf einem von dem Vorwärtsweg und dem Rückwärtsweg der Hinundherbewegung läuft, ein erstes Teil (1a6), das dem ersten Abschnitt in dem lagenartigen Element entspricht, durch Anwenden einer Menge (J/m) der Ultraschallenergie, die dem ersten Teil entspricht, geschweißt wird und ein zweites Teil (1a4), das dem zweiten Abschnitt in dem lagenartigen Element entspricht, durch Anwenden einer Menge (J/m) der Ultraschallenergie, die dem zweiten Teil entspricht, geschweißt wird.

3. Ultraschallschweißvorrichtung eines lagenartigen Elements, das mit einem absorbierenden Artikel in Verbindung steht, nach Anspruch 1, wobei
wenn das Ultraschallverarbeitungselement durch den ersten Abschnitt (A1a6) auf einem von dem Vorwärtsweg und dem Rückwärtsweg der Hinundherbewegung läuft, ein erstes Teil (1a6), das dem ersten Abschnitt in dem lagenartigen Element entspricht, durch Anwenden einer Menge (J/m) der Ultraschallenergie, die dem ersten Teil entspricht, geschweißt wird,
wenn das Ultraschallverarbeitungselement durch den zweiten Abschnitt (A1a4) auf einem anderen von dem Vorwärtsweg und dem Rückwärtsweg der Hinundherbewegung läuft, ein zweites Teil (1a4), das dem zweiten Abschnitt in dem lagenartigen Element entspricht, durch Anwenden einer Menge (J/m) der Ultraschallenergie, die dem zweiten Teil entspricht, geschweißt wird und
auf dem Vorwärtsweg das Ultraschallverarbeitungselement durch den ersten Abschnitt und danach den zweiten Abschnitt läuft und der zweite Teil (1a4) nicht in dem zweiten Abschnitt auf dem Vorwärtsweg geschweißt wird,
auf dem Rückwärtsweg das Ultraschallverarbeitungselement durch den zweiten Abschnitt und danach den ersten Abschnitt läuft und der erste Teil (1a6) nicht in dem ersten Abschnitt auf dem Rückwärtsweg geschweißt wird.

4. Ultraschallschweißvorrichtung eines lagenartigen Elements, das mit einem absorbierenden Artikel in Verbindung steht, nach einem der Ansprüche 1 bis 3, wobei
das lagenartige Element (1a) eine Vielzahl von Lagen einschließt, die in einer Dickenrichtung geschichtet sind,
das lagenartige Element eine Vielzahl von Teilen einschließt, die die Anzahl von geschichteten Lagen aufweisen, die sich voneinander unterscheidet, die Vielzahl von Teilen in der CD-Richtung angeordnet ist und
angenommen wird, dass ein Teil, das die größere Anzahl von geschichteten Lagen aufweist, in der Vielzahl von Teilen ein erstes Teil (1a6) ist und ein Teil, dass die Anzahl von geschichteten Lagen aufweist, die kleiner ist als die Anzahl davon in dem ersten Teil, ein zweites Teil (1a4) ist,
der erste Abschnitt (A1a6) dem ersten Teil entspricht und der zweite Abschnitt (A1a4) dem zweiten Teil entspricht und
eine Menge (J/m) der Ultraschallenergie, die pro Einheitslänge in dem ersten Abschnitt angewendet wird, größer ist als eine Menge (J/m) der Ultraschallenergie, die pro Einheitslänge in dem zweiten Abschnitt angewendet wird.

5. Ultraschallschweißvorrichtung eines lagenartigen Elements, das mit einem absorbierenden Artikel in Verbindung steht, nach einem der Ansprüche 1 bis 3, wobei
das lagenartige Element (1a) eine Vielzahl von Teilen einschließt, zwischen oder unter denen die Schweißnahtfestigkeit unterschiedlich voneinander zu erzeugen ist, Vielzahl von Teilen in der CD-Richtung angeordnet ist und
angenommen wird, dass ein Teil, das eine größere Schweißnahtfestigkeit aufzuweisen hat, in der Vielzahl von Teilen ein erstes Teil (1a6) ist und ein Teil, das eine Schweißnahtfestigkeit aufzuweisen hat, die kleiner ist als die Schweißnahtfestigkeit in dem ersten Teil, ein zweites Teil (1a4) ist,
der erste Abschnitt (A1a6) dem ersten Teil entspricht und der zweite Abschnitt (A1a4) dem zweiten Teil entspricht und
eine Menge (J/m) der Ultraschallenergie, die pro Einheitslänge in dem ersten Abschnitt angewendet wird, größer ist als eine Menge (J/m) der Ultraschallenergie, die pro Einheitslänge in dem zweiten Abschnitt angewendet wird.

6. Ultraschallschweißvorrichtung eines lagenartigen Elements, das mit einem absorbierenden Artikel in Verbindung steht, nach einem der Ansprüche 1 bis 5, wobei
die Ultraschallenergie regulierende Einheit eine Menge (J/m) der Ultraschallenergie, die pro Einheitslänge angewendet wird, durch Ändern einer Amplitude der Ultraschallschwingung des Horns verändert.

7. Ultraschallschweißvorrichtung eines lagenartigen Elements, das mit einem absorbierenden Artikel in Verbindung steht, nach einem der Ansprüche 1 bis 5, wobei
die Ultraschallenergie regulierende Einheit eine Menge (J/m) der Ultraschallenergie, die pro Einheitslänge angewendet wird, durch Ändern einer Größenordnung der Klemmkraft, mit der das lagenartige Element zwischen das Horn (61) und den Amboss (71) eingeklemmt wird, verändert.

8. Ultraschallschweißvorrichtung eines lagenartigen Elements, das mit einem absorbierenden Artikel in Verbindung steht, nach einem der Ansprüche 1 bis 5, wobei
die Ultraschallenergie regulierende Einheit eine Menge (J/m) der Ultraschallenergie, die pro Einheitslänge angewendet wird, durch Ändern einer Größe eines Raums (G) zwischen dem Horn (61) und dem Amboss (71) verändert.

9. Ultraschallschweißvorrichtung eines lagenartigen Elements, das mit einem absorbierenden Artikel in Verbindung steht, nach einem der Ansprüche 1 bis 5, wobei
die Ultraschallenergie regulierende Einheit eine Menge (J/m) der Ultraschallenergie, die pro Einheitslänge angewendet wird, durch Ändern eines Fahrgeschwindigkeitswerts, bei dem sich das Ultraschallverarbeitungselement in der CD-Richtung bewegt, verändert.

10. Ultraschallschweißvorrichtung eines lagenartigen Elements, das mit einem absorbierenden Artikel in Verbindung steht, nach einem der Ansprüche 1 bis 9, wobei
das Ultraschallverarbeitungselement, das eines von dem Horn (61) und dem Amboss (71) ist, ein Walzelement einschließt, das Walzelement rotierbar bereitgestellt und außerhalb in Bezug auf die äußere Umfangsfläche des rotierenden Elements angeordnet ist, und
sich das Walzelement in der CD-Richtung bewegt, während es auf einem schienenartigen Element rollt, das schienenartige Element sich in der CD-Richtung erstreckend bereitgestellt ist, dass es sich nicht relativ zur äußeren Umfangsfläche des rotierenden Elements bewegt, das schienenartige Element als das andere Ultraschallverarbeitungselement dient.

11. Ultraschallschweißvorrichtung eines lagenartigen Elements, das mit einem absorbierenden Artikel in Verbindung steht, nach einem der Ansprüche 1 bis 9, wobei
sich beide von dem Horn (61) und dem Amboss (71) in der CD-Richtung hin- und herbewegen, während das lagenartige Element (1a) zwischen das Horn und den Amboss eingeklemmt wird.

12. Ultraschallschweißverfahren, in welchem ein lagenartiges Element (1a), das mit einem absorbierenden Artikel in Verbindung steht, einem Ultraschallschweißverfahren unterzogen wird, wobei das lagenartige Element transportiert wird, während es auf eine äußere Umfangsfläche (30s) eines rotierenden Elements (30) gewickelt wird, das rotierende Element um seine Mittelachse (C30) rotiert, wobei das Verfahren Folgendes umfasst:
Rotieren einer Ultraschallverarbeitungseinheit (60) um die Mittelachse (C30) zusammen mit dem rotierenden Element (30), wobei die Ultraschallverarbeitungseinheit als Ultraschallverarbeitungselemente ein Horn (61) und einen Amboss (71), der dem Horn zugewandt ist, einschließt;
Bewirken, dass das Horn (61) mit Ultraschall vibriert; und
Bewirken, dass sich mindestens ein Ultraschallverarbeitungselement von dem Horn und dem Amboss in einer CD-Richtung in Bezug auf ein Teil des lagenartigen Elements hin- und herbewegt, wobei die CD-Richtung eine Transportrichtung des lagenartigen Elements überkreuzt, während das lagenartige Element zwischen dem Horn und dem Amboss eingeklemmt ist, das Teil um das rotierende Element gewickelt wird,
wobei die äußere Umfangsfläche (30s) des rotierenden Elements (30) einen ersten Abschnitt (A1a6) und einen zweiten Abschnitt (A1a4) an Positionen, die sich in der CD-Richtung voneinander unterscheiden, einschließt,
**dadurch gekennzeichnet, dass** eine Menge (J/m) an Ultraschallenergie, die pro Einheitslänge in der CD-Richtung angewendet wird, wenn das Ultraschallverarbeitungselement durch den ersten Abschnitt (A1a6) auf einem von einem Vorwärtsweg und einem Rückwärtswegs der Hinundherbewegung läuft, verschieden erzeugt wird von einer Menge (J/m) an Ultraschallenergie, die pro Einheitslänge in der CD-Richtung angewendet wird, wenn das Ultraschallverarbeitungselement durch den zweiten Abschnitt (A1a4) auf einem von dem Vorwärtsweg und dem Rückwärtsweg läuft.

## Revendications

1. Appareil de soudage par ultrasons (20) qui effectue un processus de soudage par ultrasons sur un élément en forme de feuille (1a), l'élément en forme de feuille étant associé à un article absorbant, l'élément en forme de feuille étant transporté tout en étant enroulé sur une face circonférentielle extérieure (30s) d'un élément rotatif (30), l'élément rotatif tournant autour de son axe central (C30), l'appareil comportant :
l'élément rotatif (30) ; et
une unité de traitement par ultrasons (60)
qui tourne autour de l'axe central (C30) conjointement avec l'élément rotatif, et
qui comprend, comme éléments de traitement par ultrasons,
un émetteur d'ultrasons (61) qui vibre de manière ultrasonore et
une enclume (71), l'élément en forme de feuille étant pris en sandwich entre celle-ci et l'émetteur d'ultrasons
au moins un quelconque élément de traitement par ultrasons parmi l'émetteur d'ultrasons et l'enclume étant guidé de manière à effectuer un mouvement de va-et-vient dans une direction allant dans le sens travers, la direction allant dans le sens travers croisant une direction allant dans le sens du transport de l'élément en forme de feuille,
ledit au moins un quelconque élément de traitement par ultrasons parmi l'émetteur d'ultrasons et l'enclume effectuant un mouvement de va-et-vient dans la direction allant dans le sens travers par rapport à une partie de l'élément en forme de feuille, la partie étant enroulée autour de l'élément rotatif,
la face circonférentielle extérieure (30s) de l'élément rotatif (30) comprenant une première section (A1a6) et une deuxième section (A1a4) au niveau de positions différentes les unes par rapport aux autres dans la direction allant dans le sens travers,
**caractérisé en ce que** l'appareil comporte par ailleurs une unité de régulation d'énergie ultrasonore,
l'unité de régulation d'énergie ultrasonore réalisant
une quantité (J/m) d'énergie ultrasonore appliquée par unité de longueur dans la direction allant dans le sens travers quand l'élément de traitement par ultrasons passe au travers de la première section sur l'un quelconque parmi un chemin d'avance et un chemin de retour du mouvement de va-et-vient
différente par rapport à
une quantité (J/m) d'énergie ultrasonore appliquée par unité de longueur dans la direction allant dans le sens travers quand l'élément de traitement par ultrasons passe au travers de la deuxième section sur l'un quelconque parmi le chemin d'avance et le chemin de retour.

2. Appareil de soudage par ultrasons d'un élément en forme de feuille associé à un article absorbant selon la revendication 1, dans lequel
quand l'élément de traitement par ultrasons passe au travers de la première section (A1a6) et de la deuxième section (A1a4) sur l'un quelconque parmi le chemin d'avance et le chemin de retour du mouvement de va-et-vient, une première partie (1a6) correspondant à la première section dans l'élément en forme de feuille est soudée par l'application d'une quantité (J/m) de l'énergie ultrasonore correspondant à la première partie, et une deuxième partie (1a4) correspondant à la deuxième section dans l'élément en forme de feuille est soudée par l'application d'une quantité (J/m) de l'énergie ultrasonore correspondant à la deuxième partie.

3. Appareil de soudage par ultrasons d'un élément en forme de feuille associé à un article absorbant selon la revendication 1, dans lequel
quand l'élément de traitement par ultrasons passe au travers de la première section (A1a6) sur l'un parmi le chemin d'avance et le chemin de retour du mouvement de va-et-vient, une première partie (1a6) correspondant à la première section dans l'élément en forme de feuille est soudée par l'application d'une quantité (J/m) de l'énergie ultrasonore correspondant à la première partie,
quand l'élément de traitement par ultrasons passe au travers de la deuxième section (A1a4) sur un autre parmi le chemin d'avance et le chemin de retour du mouvement de va-et-vient, une deuxième partie (1a4) correspondant à la deuxième section dans l'élément en forme de feuille est soudée par l'application d'une quantité (J/m) de l'énergie ultrasonore correspondant à la deuxième partie, et
sur le chemin d'avance, l'élément de traitement par ultrasons passe au travers de la première section et ensuite au travers de la deuxième section, et la deuxième partie (1a4) n'est pas soudée dans la deuxième section sur le chemin d'avance,
sur le chemin de retour, l'élément de traitement par ultrasons passe au travers de la deuxième section et ensuite au travers de la première section, et la première partie (1a6) n'est pas soudée dans la première section sur le chemin de retour.

4. Appareil de soudage par ultrasons d'un élément en forme de feuille associé à un article absorbant selon l'une quelconque des revendications 1 à 3, dans lequel
l'élément en forme de feuille (1a) comprend une pluralité de feuilles empilées dans une direction allant dans le sens de l'épaisseur,
l'élément en forme de feuille comprend une pluralité de parties ayant le nombre de feuilles empilées différentes les unes par rapport aux autres, les parties de la pluralité de parties étant agencées dans la direction allant dans le sens travers, et
en supposant qu'une partie ayant le plus grand nombre de feuilles empilées dans la pluralité de parties est une première partie (1a6), et qu'une partie ayant le nombre de feuilles empilées inférieur au nombre de celles-ci dans la première partie est une deuxième partie (1a4),
la première section (A1a6) correspond à la première partie, et la deuxième section (A1a4) correspond à la deuxième partie, et
une quantité (J/m) de l'énergie ultrasonore appliquée par unité de longueur dans la première section est supérieure à une quantité (J/m) de l'énergie ultrasonore appliquée par unité de longueur dans la deuxième section.

5. Appareil de soudage par ultrasons d'un élément en forme de feuille associé à un article absorbant selon l'une quelconque des revendications 1 à 3, dans lequel
l'élément en forme de feuille (1a) comprend une pluralité de parties entre lesquelles ou parmi lesquelles la résistance de la soudure est destinée à être rendue différente d'une partie à une autre, les parties de la pluralité de parties étant agencées dans la direction allant dans le sens travers, et
en supposant qu'une partie qui est destinée à avoir une plus grande résistance de la soudure dans la pluralité de parties est une première partie (1a6), et qu'une partie qui est destinée à avoir une résistance de la soudure qui est plus faible par rapport à la résistance de la soudure dans la première partie est une deuxième partie (1a4),
la première section (A1a6) correspond à la première partie, et la deuxième section (A1a4) correspond à la deuxième partie, et
une quantité (J/m) de l'énergie ultrasonore appliquée par unité de longueur dans la première section est supérieure à une quantité (J/m) de l'énergie ultrasonore appliquée par unité de longueur dans la deuxième section.

6. Appareil de soudage par ultrasons d'un élément en forme de feuille associé à un article absorbant selon l'une quelconque des revendications 1 à 5, dans lequel
l'unité de régulation d'énergie ultrasonore change une quantité (J/m) de l'énergie ultrasonore appliquée par unité de longueur, en changeant une amplitude des vibrations ultrasonores de l'émetteur d'ultrasons.

7. Appareil de soudage par ultrasons d'un élément en forme de feuille associé à un article absorbant selon l'une quelconque des revendications 1 à 5, dans lequel
l'unité de régulation d'énergie ultrasonore change une quantité (J/m) de l'énergie ultrasonore appliquée par unité de longueur, en changeant une intensité de la force de prise en sandwich avec laquelle l'élément en forme de feuille est pris en sandwich entre l'émetteur d'ultrasons (61) et l'enclume (71).

8. Appareil de soudage par ultrasons d'un élément en forme de feuille associé à un article absorbant selon l'une quelconque des revendications 1 à 5, dans lequel
l'unité de régulation d'énergie ultrasonore change une quantité (J/m) de l'énergie ultrasonore appliquée par unité de longueur, en changeant une taille d'un espace (G) entre l'émetteur d'ultrasons (61) et l'enclume (71).

9. Appareil de soudage par ultrasons d'un élément en forme de feuille associé à un article absorbant selon l'une quelconque des revendications 1 à 5, dans lequel
l'unité de régulation d'énergie ultrasonore change une quantité (J/m) de l'énergie ultrasonore appliquée par unité de longueur, en changeant une valeur de vitesse d'avance à laquelle l'élément de traitement par ultrasons se déplace dans la direction allant dans le sens travers.

10. Appareil de soudage par ultrasons d'un élément en forme de feuille associé à un article absorbant selon l'une quelconque des revendications 1 à 9, dans lequel
l'élément de traitement par ultrasons qui est l'un quelconque parmi l'émetteur d'ultrasons (61) et l'enclume (71) comprend un élément formant rouleau, l'élément formant rouleau étant mis en oeuvre de manière rotative et agencé à l'extérieur par rapport à la face circonférentielle extérieure de l'élément rotatif, et
l'élément formant rouleau se déplace dans la direction allant dans le sens travers tout en roulant sur un élément en forme de rail, l'élément en forme de rail étant mis en oeuvre de manière à s'étendre dans la direction allant dans le sens travers pour ne pas se déplacer par rapport à la face circonférentielle extérieure de l'élément rotatif, l'élément en forme de rail servant comme étant l'autre élément de traitement par ultrasons.

11. Appareil de soudage par ultrasons d'un élément en forme de feuille associé à un article absorbant selon l'une quelconque des revendications 1 à 9, dans lequel
à la fois l'émetteur d'ultrasons (61) et l'enclume (71) se déplacent en un mouvement de va-et-vient dans la direction allant dans le sens travers alors que l'élément en forme de feuille (1a) est pris en sandwich entre l'émetteur d'ultrasons et l'enclume.

12. Procédé de soudage par ultrasons dans lequel un élément en forme de feuille (1a) associé à un article absorbant est soumis à un processus de soudage par ultrasons, l'élément en forme de feuille étant transporté tout en étant enroulé sur une face circonférentielle extérieure (30s) d'un élément rotatif (30), l'élément rotatif tournant autour de son axe central (C30), le procédé comportant les étapes consistant à :
faire tourner une unité de traitement par ultrasons (60) autour de l'axe central (C30) conjointement avec l'élément rotatif (30), l'unité de traitement par ultrasons comprenant, comme éléments de traitement par ultrasons, un émetteur d'ultrasons (61) et une enclume (71) orientée vers l'émetteur d'ultrasons ;
amener l'émetteur d'ultrasons (61) à vibrer de manière ultrasonore ; et
amener au moins un quelconque élément de traitement par ultrasons parmi l'émetteur d'ultrasons et l'enclume à effectuer un mouvement de va-et-vient dans une direction allant dans le sens travers par rapport à une partie de l'élément en forme de feuille, la direction allant dans le sens travers croisant une direction allant dans le sens du transport de l'élément en forme de feuille, alors que l'élément en forme de feuille est pris en sandwich entre l'émetteur d'ultrasons et l'enclume, la partie étant enroulée autour de l'élément rotatif,
la face circonférentielle extérieure (30s) de l'élément rotatif (30) comprenant une première section (A1a6) et une deuxième section (A1a4) au niveau de positions différentes les unes par rapport aux autres dans la direction allant dans le sens travers,
**caractérisé en ce qu'**une quantité (J/m) d'énergie ultrasonore appliquée par unité de longueur dans la direction allant dans le sens travers quand l'élément de traitement par ultrasons passe au travers de la première section (A1a6) sur l'un quelconque parmi un chemin d'avance et un chemin de retour du mouvement de va-et-vient différente par rapport à une quantité (J/m) d'énergie ultrasonore appliquée par unité de longueur dans la direction allant dans le sens travers quand l'élément de traitement par ultrasons passe au travers de la deuxième section (A1a4) sur l'un quelconque parmi le chemin d'avance et le chemin de retour.
